# EUROPEAN PATENT SPECIFICATION

(11) **EP 1 841 514 B1**
(45) Date of publication and mention of the grant of the patent: **17.04.2013**
(21) Application number: 05850747.6
(22) Date of filing: 22.12.2005
(51) Int. Cl.: B01D 29/60

(54) **DEVICE FOR DETECTING CONDITIONS OF A FLUID, IN PARTICULAR LUBRICATING OIL, USED ON VEHICLES IN COMBINATIONS WITH A FILTER**
VORRICHTUNG ZUR ERFASSUNG VON ZUSTÄNDEN EINES FLUIDS, INSBESONDERE SCHMIERÖL, ZUR VERWENDUNG BEI FAHRZEUGEN IN KOMBINATIONEN MIT EINEM FILTER
DISPOSITIF POUR DETECTER DES ETATS D'UN FLUIDE, EN PARTICULIER D'UNE HUILE LUBRIFIANTE, UTILISE DANS DES VEHICULES EN ASSOCIATION AVEC UN FILTRE

(30) Priority: 24.12.2004 IT TO20040904
(43) Date of publication of application: 10.10.2007
(73) Proprietor: ELTEK S.p.A., I-15033 Casale Monferrato (Alessandria) (IT)
(72) Inventor: GADINI, Costanzo, I-15040 Frassineto Po (IT)
(74) Representative: Gallarotti, Franco
(86) International application number: PCT/IB2005/004016
(87) International publication number: WO 2006/067625

(56) References cited:
- WO-A-2004/053304
- DE-A1- 10 202 030
- US-A- 4 626 344
- US-A1- 2003 046 985
- US-A1- 2004 231 402

## Description

### TEXT OF THE DESCRIPTION

The present invention relates to a device for detecting conditions of a fluid, in particular lubricating oil used on vehicles in combination with a filter.

Correct operation of a lubrication circuit, for example, of an internal-combustion engine of a motor vehicle, presupposes that the oil used will maintain a good quality. The characteristics of the oil tend, however, to degrade over time, on account of contamination of various nature; for example, in the specific case of internal-combustion engines, fumes, unburnt particles, metallic residue, and fuel can come occasionally into contact with the lubricating oil of the engine, so reducing its operating characteristics.

For the purpose of preserving the quality of the oil there are thus provided purposely designed filters, operative for withholding the aforesaid contaminant elements. Notwithstanding the presence of a filter, it may, however, happen that the characteristics of the oil undergo degradation before the expiration date normally recommended for its replacement, and it is clear that the premature decay of the quality of the oil can be the cause of serious damage to the lubrication system or to the engine. On the other hand, it is also possible that, at the moment of the programmed replacement, the oil will still conserve excellent characteristics, which would render replacement thereof momentarily superfluous.

For the aforesaid reasons, purposely designed detection devices have been proposed, aimed at monitoring one or more qualitative characteristics of the lubricating oil. When said devices detect the decay of a certain characteristic with respect to a pre-determined threshold value, an appropriate control system sees to signalling the fault, in order to indicate the need to proceed to oil replacement. Said devices usually comprise sensors equipped with electrodes designed for contact with the fluid or oil, between which is, for example, applied and/or detected a difference of potential, a voltage, or a signal, for the purposes of detection and/or measurement of quantities such as acidity, conductivity, resistivity or capacitance of the oil, from which quantities it is possible to deduce some characteristics of interest regarding the oil itself.

In certain cases, the detection device is designed for being positioned in a specific installation seat, which necessarily implies a specific pre-arrangement of the engine of the vehicle, which must be equipped with the aforesaid seat, designed for enabling housing and hydraulic connection of the device. On account of the different structures or conformations of the vehicle engines, the aforementioned devices and the respective seats are typically shaped so as to be able to be housed in areas available for the purpose in the engine compartment, said areas being frequently difficult to access for the purposes of maintenance, for example, for periodic cleaning.

In other known solutions, the detection device is, instead, directly integrated in the oil filter, with the consequence that the replacement of the filter, when occluded or clogged, determines also the replacement of the device. Furthermore, irrespective of said disadvantage, said solution presents the drawback that the detection device must be made in a small space on account of the limitations imposed by the by now consolidated versions of filters, which have substantially standardized structures and dimensions. This involves in general the construction of sensors having complex shapes, low reliability and/or high production costs.

WO 2004/053304 A, upon which the preamble of claim 1 is based, discloses a subsidiary device to be installed between an oil filter and an oil pump. The device has an housing within which an impeller and a heating coil are installed.

US 4626344 A discloses an oil filter restriction sensor device for warning the operator of an internal combustion engine when the engine oil filter has become restricted and requires replacement.

In its general terms, the present invention proposes solving one or more of the aforesaid drawbacks. This and other purposes still, which will clearly emerge from what follows, are achieved according to the present invention by a device for detecting characteristics of a fluid, in particular lubricating oil, used on vehicles in combination with a filter, said device having the characteristics indicated in the annexed claims, which are understood as forming an integral part of the present description.

Further purposes, characteristics and advantages of the present invention will clearly emerge from the ensuing detailed description and from the annexed drawings, which are provided purely by way of explanatory and non-limiting example and in which:
- Figure 1 is a schematic plan view of a detection device not according to the invention;
- Figure 2 is a schematic cross-sectional view according to the line II-II of Figure 1;
- Figure 3 is a schematic cross-sectional view similar to that of Figure 1, with the device in a first possible condition of use;
- Figure 4 is a schematic cross-sectional view similar to that of Figure 1, with the device in a second possible condition of use;
- Figure 5 is a schematic cross-sectional view according to the line V-V of Figure 2, aimed at illustrating sensor means of the device;
- Figure 6 is an exploded view of two details of Figure 5;
- Figure 7 is a schematic cross-sectional view of a first variant of the sensor means illustrated in Figure 5;
- Figure 8 is an exploded cross-sectional view of three details of Figure 7;
- Figure 9 is a schematic cross-sectional view of a second variant of the sensor means illustrated in Figure 5;
- Figure 10 is a schematic cross-sectional view of a third variant of the sensor means illustrated in Figure 5;
- Figure 11 is a schematic cross-sectional view of three details of Figure 10;
- Figure 12 is a schematic cross-sectional view of three further details of Figure 10;
- Figure 13 is a schematic cross-sectional view of a possible second embodiment of a detection device not according to the invention, equipped with sensor means built in accordance with a fourth variant;
- Figure 14 is a schematic cross-sectional view according to the line XIV-XIV of Figure 13;
- Figure 15 is a schematic cross-sectional view of a fifth variant of the sensor means illustrated in Figure 5;
- Figure 16 is a schematic cross-sectional view of a detail of the sensor means illustrated in Figure 15;
- Figure 17 is a schematic cross-sectional view of a possible third embodiment of a detection device not to the invention;
- Figure 18 is a schematic plan view of a detection device in accordance with a possible fourth embodiment not according to the invention;
- Figure 19 is a schematic cross-sectional view, aimed at illustrating a possible embodiment of sensor means of the device illustrated in Figure 18;
- Figure 20 is a schematic cross-sectional view of four details of the sensor means illustrated in Figure 19;
- Figure 21 is a schematic cross-sectional view (and according to the line XXI-XXI of Figure 22) of a detection device according to the invention, equipped with sensor means built in accordance with a further possible variant;
- Figure 22 is a schematic cross-sectional view according to the line XXII-XXII of Figure 21;
- Figure 23 is a schematic cross-sectional view of the device illustrated in Figure 21, represented rotated axially through 90° with respect to the latter (and according to the line XXIII-XXIII of Figure 24);
- Figure 24 is a schematic cross-sectional view according to the line XXIV-XXTV of Figure 23;
- Figures 25 and 26 are schematic cross-sectional views, similar to those of Figures 21 and 23, with the device according to the invention in a possible condition of use;
- Figure 27 is an exploded schematic cross-sectional view of a casing of a detection device not according to the invention;
- Figure 28 is an exploded schematic cross-sectional view of the detection device comprising the casing illustrated in Figure 27;
- Figure 29 is a schematic cross-sectional view of the detection device illustrated in Figure 28;
- Figure 30 is an exploded schematic cross-sectional view of some parts of a detection device according to a possible embodiment of the invention;
- Figure 31 is a schematic cross-sectional view of a lid of the device illustrated in Figure 30, represented rotated axially through 90° with respect to the latter figure;
- Figure 32 is a schematic cross-sectional view of the device comprising the parts illustrated in Figure 30;
- Figure 33 is a schematic cross-sectional view of the device comprising the parts illustrated in Figure 30, represented rotated axially through 90° with respect to Figure 32;
- Figure 34 is a schematic cross-sectional view of a first sensor with electrodes made of conductive synthetic material, which can be used in a device according to the invention;
- Figure 35 is a schematic cross-sectional view of a second sensor with electrodes formed at least in part with conductive synthetic material, which can be used in a device according to the invention;
- Figure 36 is a schematic cross-sectional view of a third sensor with electrodes formed at least in part with conductive synthetic material, which can be used in a device according to the invention;
- Figure 37 is a schematic cross-sectional view of a component of a detection device equipped with sensor means which have electrodes formed at least in part with moulded conductive synthetic material;
- Figure 38 is a schematic cross-sectional view according to the line XXXVIII-XXXVIII of Figure 37; and
- Figures 39 and 40 are schematic cross-sectional views aimed at illustrating the principle of use of a generic moulding apparatus that can be used for the purposes of obtaining the component illustrated in Figures 37-38.

The idea underlying the present invention is to provide a detection device having a structure designed to be set, if desired, between an ordinary or normal filter for a fluid circulating in a vehicle and the respective part or joint of the vehicle on which said filter is to be normally connected. The aforesaid filter can be, for example, the one provided for purification of the lubricating oil of an internal-combustion engine, which in use is normally connected mechanically and hydraulically to a part of the engine, in particular via a hydraulic joint comprising an internal conduit, equipped with threaded joint, and a peripheral conduit, provided with sealing means, the internal conduit and the peripheral one being designed, respectively, to cause the oil that is to be filtered to flow towards the filter and then the filtered oil to flow away towards the engine.

The structure of the device according to the invention is preferably made in such a way as to comprise at least one respective first joint, for mechanical and hydraulic coupling to the aforesaid joint of the filter, and at least one respective second joint, for mechanical and hydraulic coupling to the aforesaid internal and peripheral conduits of the engine.

The aforesaid first and second hydraulic joints of the device according to the invention are preferably complementary to one another, and in particular a male joint and a female joint, respectively, of one and the same type, and of the type commonly used on filters normally available on the market. The first and the second joints are preferably arranged axially with respect to one another; preferably, at least part of said first and second joints is formed in a position corresponding to a respective plane, the two planes being parallel to, or equidistant from, one another, and preferably substantially coincident with two opposite faces of the device according to the invention.

The device according to the invention can in this way be mounted on a part of a vehicle, hereinafter defined generically as "engine", in a position corresponding to the respective joint on which a filter of the type commonly available on the market is to be normally connected, with the latter that is, instead, connected on the device, which thus functions also as support and/or joint for the filter.

This enables the detection device according to the invention to be installed or not, according to the need. When originally provided on the vehicle, set between the filter and the engine, the detection device can be removed, if need be, for then re-installing just the filter directly on the engine. Alternatively, the detection device can be conveniently mounted at a later date on an engine to which only the filter is originally associated, as commonly provided in all vehicles. In this second case, the device will be preferably equipped with a respective control circuit, in particular of an electronic and independent type, i.e., dedicated to the device itself. In this case, the electronic control circuit can be integrated directly in the detection device forming the subject of the present invention. The control circuit of the device could, on the other hand, be integrated, either in all or in part, in a control unit already present on the vehicle, equipped with means of interfacing and/or of interconnection to the electrical and/or electronic part of the device itself. The operating program of said unit can, if need be, be of an updateable or re-programmable type, in particular for enabling control of the device according to the invention.

The device is designed for detecting the quality of the fluid that has already been filtered. Advantageously, the device according to the invention can integrate also further sensor means, in particular a pressure sensor, aimed at monitoring the state of efficiency of the filter, for example, for detecting its possible conditions of clogging, and/or a temperature sensor, for example, for correlating the measurements to the variations in temperature of the fluid and/or of the environment.

In particularly advantageous embodiments, the structure of the detection device according to the invention is of a modular or adaptable type, in order to allow to obtain, via a limited number of basic components, a multiplicity of different versions of the device itself, or, for adapting a same basic structure of the detecting device to a multiplicity of different filters and/or engines.

As previously mentioned, the first and the second joints are of a type compatible with the ones present, respectively, on the vehicle and on the filter, the two joints being preferably of one and the same type. On the other hand, in possible variant embodiments or applications of the invention, the device could function not only as a detector of characteristics of the fluid, but also as an adapter, for enabling installation, on a vehicle, of a filter available on the market but not originally envisaged for that type of vehicle. In such a perspective, the two joints of the device may also be of types different from one another, it being understood that the first joint will be designed for connection to the vehicle and the second joint will be designed for the particular filter.

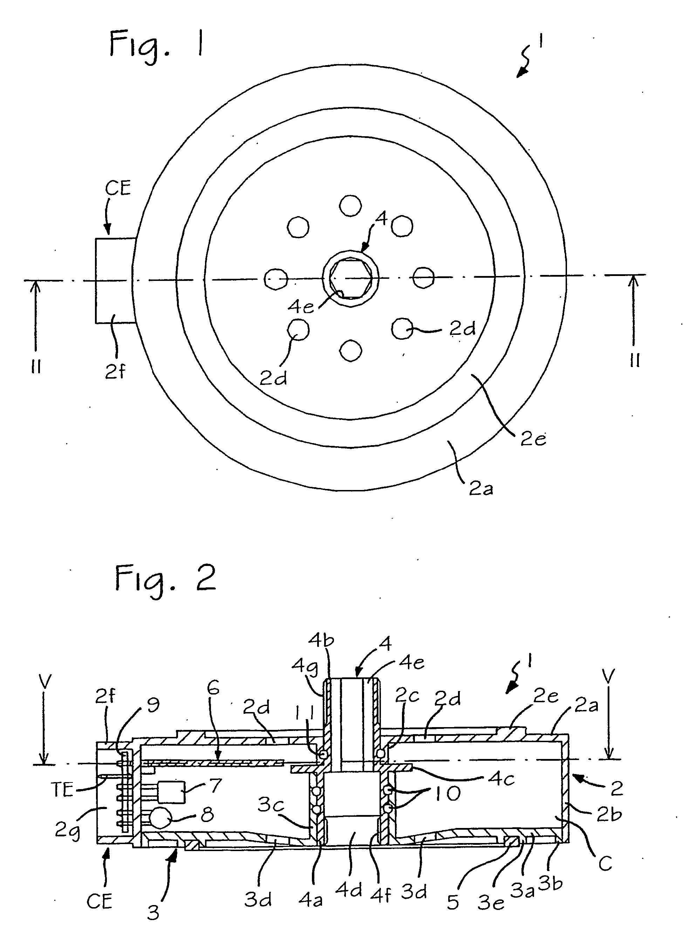

In Figures 1 to 6 is represented a first possible embodiment of a detection device not according to the invention, designated as a whole by 1. The device 1 comprises a casing constituted by a main body 2, closed at one end via an end body or lid 3. The main body 2 and the lid 3 are coupled to one another in a sealed way, in the regions of mutual joining, with modalities in themselves known. Joining could, for example, be obtained via laser welding, or by thermofusion or via hot blade, or again via means suitable for determining melting and/or mixing of at least part of the material constituting the main body 2 and the lid 3. Said solution presents the advantage of not requiring necessarily the presence of a sealing element in the joining region. Alternatively, joining could occur via mechanical-coupling means of a female-thread/male-thread type or via mechanical couplings (such as ones of the press-block type, bayonet coupling, snap-action coupling, etc.). In this case, there are advantageously provided additional sealing means, mounted in a position corresponding to the joining region, such as, for example, gaskets made of thermoplastic material or of an O-ring type. The casing of the device 1 could also be made up of a larger number of parts as compared to the case illustrated (for example, with two end lids), and hence the joining regions could be in a larger number and/or located in different areas, and/or using combinations of the aforesaid technologies of fixing and sealing.

The body 2 comprises an end wall 2a, from which there branch off orthogonally a peripheral wall 2b, preferably cylindrical, and an internal wall 2c, which is also preferably cylindrical, the height and the diameter of the peripheral wall 2b being greater than those of the internal wall 2c. The end wall 2a has a central passage, in a position corresponding to which the wall 2c is formed, which in this way defines a first portion of a positioning seat for a connection element 4 of the device 1, referred to hereinafter as connection sleeve, which substantially forms a central pipe of the device 1, as well as part of the respective hydraulic and mechanical connection element, as will emerge from what follows. The walls 2b and 2c are substantially concentric or equidistant from one another, with the respective central axes preferably coincident with one another and with the axis of the device 1. In the non-limiting example provided, the walls 2b and 2c rise in one and the same direction in order to reduce the overall dimensions of the device; said direction is preferably referred to the end wall 2a, and parallel to the axis of the device 1, or of the aforesaid positioning seat or of the sleeve 4. In the end wall 2a there are moreover present lateral passages or holes 2d, arranged substantially according to a circumference about the central hole delimited by the wall 2c; the region in which the holes 2d are formed is surrounded by a relief 2e of annular profile, or with other closed profile, which rises from the wall 2a towards the outside of the device 1 and forms part of a sealing system. In a variant (not represented), the relief 2e could be formed by an element independent from the body 2, such as an annular or closed-profile element, preferably made of elastic material, such as, for example, an O-ring, or other suitably designed sealing element. The body 2 is shaped so as to form at least part of an electrical connector CE; for this purpose, the body 2 comprises a side portion 2f, which extends in a radial direction from the peripheral wall 2b, forming a cavity or hollow 2g. The side portion 2f is preferably provided with engagement appropriate means, in particular for a complementary connector (not represented) forming part of an external wiring to which the device 1 is to be connected. In another variant (not represented), the side portion 2f could be configured as an element independent of the body 2, designed to be fixed to the latter (for example via couplings, welding, bonding or other known technique).

Also the lid 3 comprises a respective end wall 3a, from which there branch off orthogonally a peripheral wall 3b, preferably cylindrical, and an internal wall 3c, also preferably cylindrical, the wall 3b having a larger diameter and a smaller height with respect to the wall 3c. The end wall 3a has a central passage, in a position corresponding to which the wall 3c is formed, which in this way defines a second portion of the aforesaid positioning seat for the connection sleeve 4. Also the walls 3b and 3c are substantially concentric or equidistant from one another, with the respective central axes coincident with one another and with the axis of the device 1. In the non-limiting example provided, the walls 3b and 3c extend in elevation in directions opposite with respect to the end wall 3a. There is nothing to rule out that, as in the case of the body 2, said direction should be substantially the same, in particular for the purpose of reducing the overall dimensions of the device 1, said direction being preferably parallel to the axis of the device 1, or of the aforesaid positioning seat, or of the sleeve 4. As may be appreciated, the seat portions defined by the central walls 2c and 3c of the main body 2 and of the lid 3, respectively, are substantially coaxial with respect to one another. In the wall 3a of the lid there are moreover present side passages or holes 3d, arranged substantially according to a circumference, about the central hole delimited by the wall 3c; the region in which the holes 3d are formed is surrounded by a relief 3e, having an annular or in any case closed-profile shape, which rises from the wall 3a, towards the outside of the device 1. In the case exemplified, the annular relief 3e comprises two walls that are concentric with respect to one another, which define a positioning seat for a sealing element or gasket 5, having a substantially annular shape and with quadrangular cross section. The mean diameter of the gasket 5, or of the relief 3e defining its seat, is substantially similar to the mean diameter of the annular relief 2e present on the wall 2a of the body 2.

Within the cavity delimited by the body 2 and by the lid 3, designated by C, sensor means are housed, which, in the case exemplified, comprise at least the electrodes of a sensor 6 for detecting one or more electrical or physical quantities, for example, conductivity, resistivity, capacitance, impedance, a temperature sensor 7 and/or a quartz oscillator 8. The connection terminals of said sensor means are inserted through respective passages formed in the peripheral wall 2b in a position corresponding to the aforesaid hollow 2g, with the respective ends that project into the hollow itself, where they are electrically and mechanically connected to an electrical-connection support, designated by 9, comprising, for example, a printed circuit. Said printed circuit 9 can form part of an electronic measurement and/or control circuit of the device 1, or else part of a circuit for interconnection of the aforesaid sensor means. In order to guarantee the necessary seal, said passages and/or said electronic circuit are appropriately sealed, for example, using suitable resins or glues. Alternatively, the body 2 could be made by overmoulding the plastic material directly on at least part of said elements, i.e., at least on the electrical terminals of the sensor means 6-8 or on other electrical terminals to which said sensor means 6-8 are subsequently connected in the course of production of the device 1. The electrical terminals of the sensor means 6-8 could moreover be provided with respective means of coating and/or protection, or else be insulated, for example, against possible phenomena of corrosion due to contact with the fluid to be detected. Connected to said terminals and/or to the support 9 are electrical connection elements TE, which, together with the portion of body 2f, form the electrical connector CE, for example, of a male type, suitable for coupling with a complementary connector, for example, of a female type (not represented), which is in turn connected to a respective wiring and/or to a control unit of the vehicle. Preferably, between the aforesaid connectors, sealing means are provided, in order to prevent any infiltration of moisture and dirt into the hollow 2g.

The connection sleeve 4 comprises a hollow body having a basically cylindrical shape, with a lower portion 4a and an upper portion 4b, the former having an external diameter greater than the latter. In the embodiment of the device 1 exemplified herein, the body of the sleeve 4 has an intermediate portion in relief or flange-like portion, designated by 4c, projecting from the body of the sleeve itself, substantially in a position corresponding to the area of union between the two portions 4a and 4b. The flange-like portion 4c enables exertion of a thrust or compression at least on the lid 3, when the device 1 is mounted for use. Said thrust on the lid 3 is consequently transferred also onto the body 2, via the joining region between the two elements 2 and 3, said region being preferably made with a structure designed for said purpose, for example, via appropriate reliefs or couplings designed to transmit the thrust. The sleeve 4 has internally a conduit, having a respective lower stretch 4d, corresponding to the portion 4a, and an upper stretch 4e, corresponding to the portion 4b. The sleeve 4 is preferably coupled to the assembly formed by the body 2 and the lid 3 so as to be able to rotate about its own axis. For said purpose, as may be appreciated in Figure 2, the lower portion 4a is designed to be housed in the seat portion or passage formed by the internal wall 3c of the lid 3, the upper portion 4b is designed to be partially housed in the seat portion or passage formed by the internal wall 2c of the body 2, and the intermediate portion 4c is positioned between the facing ends of said internal walls 2c, 3c. Between the bottom portion 4a of the sleeve and the wall 3c there are sealing means 10, in the form of rings of an O-ring type. At least one similar sealing element 11 operates between the top portion 4b of the sleeve and the wall 2c. The sealing elements 10 and 11 are operative for preventing any leakage of fluid towards the housing seat of the sleeve 4.

The two longitudinal ends of the sleeve 4 are equipped with respective mechanical and hydraulic coupling means. Said means comprise, in the case exemplified, a female thread 4f, defined inside the lower portion 4a, and a male thread 4g, defined on the outside of the upper portion 4b. The coupling means 4f, 4g are complementary to one another, or of the same type, but, respectively, of a female type and of a male type, in the case exemplified, or in any case of the type designed to enable in theory mutual coupling thereof. The conduit upper stretch 4e is preferably shaped so as to provide the seat for a tool, such as, for example, a spanner of the Allen type, designed to enable the assembly of the device 1 on an apparatus requiring use thereof, such as a vehicle, as will emerge from what follows. For this purpose, the conduit upper stretch 4e can have a substantially hexagonal cross section.

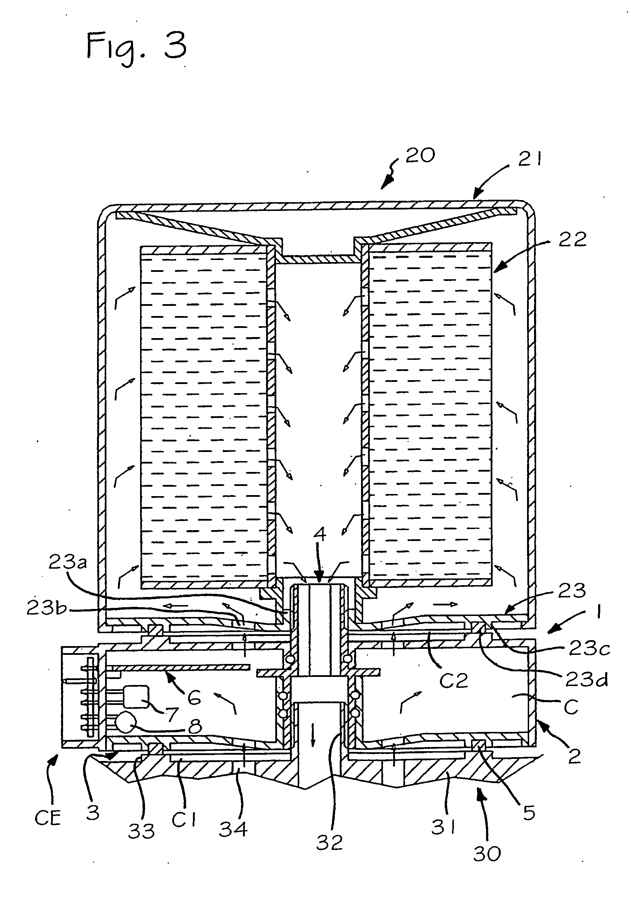

In Figure 3 the device 1 is illustrated in a condition of possible use, i.e., set between a filter 20 and a user apparatus 30, such as the engine of a vehicle. The engine 30, of a type in itself known, comprises an end wall 31, from which there rise a substantially cylindrical conduit 32 and an annular relief 33, where, in the region comprised between the conduit 32 and the relief 33, the end wall 31 has a series of passages or holes 34. On the outer part of the conduit 32 a thread is present, so as to form a threaded joint of a male type, which is complementary to the thread 4f defined within the bottom portion 4a of the sleeve 4 (see Figure 1). The mean diameter of the annular relief 33 is in the order of the mean diameter of the gasket 5 of the device 1 in such a way that, in the installed condition, the latter operates in a sealed way with respect to the former. In said installed condition, in the female attachment of the device 1, constituted by the internal threaded part 4f of the sleeve 4 (see Figure 2), there is screwed the male attachment constituted by the threaded outer wall of the conduit 32, so as to provide a mechanical and hydraulically sealed fixing between the device 1 and the engine 30. In said condition of assembly, between the engine 30 and the device 1 a passage chamber is formed, designated by C1, delimited between at least part of the outside of the lid 3, part of the outside of the wall 31 of the engine 30, part of the internal portion of the gasket 5, and part of the outer portion of the conduit 32. Said chamber C1 is in communication with the passages 34 of the engine and 3d of the device 1.

The filter 20, which is of a type in itself known, comprises a main body 21, which houses a filter element 22 and is sealed at the bottom via a further body or end element 23. Said end element 23 has a central hole, in a position corresponding to which there rises a respective internal wall 23a, which is substantially cylindrical and equipped internally with a thread so as to form a threaded attachment of a female type, which is complementary to the thread of a male type 4g defined on the outside of the upper portion 4b of the sleeve 4 (see Figure 2). In the end element 23 there is also present a series of passages or holes 23b, surrounded by an annular relief 23c, facing the outside of the filter 20, said annular relief 23c being in particular provided with two mutually concentric reliefs. The relief 23c defines a seat for a respective sealing element or gasket 23d, which has a substantially annular shape and a rectangular cross section. The mean diameter of the gasket 23d is in the order of the mean diameter of the annular relief 2e present on the end wall 2a of the body 2, so that, in the mounted condition of the device 1, the gasket 23d acts as a seal for the annular relief 2e. In said installed condition, the male joint of the device 1, constituted by the threaded part 4g of the sleeve 4, is screwed into the female joint, constituted by the internally threaded wall 23a of the filter 20, so as to provide a mechanical and hydraulically sealed fixing between the device 1 and the filter 20. In said condition of assembly, between the device 1 and the filter 20 a chamber of passage is formed, designated by C2, delimited between at least the outside of the body 2, part of the outside of the end element 23 of the filter 20, part of the internal portion of the gasket 23d, and part of the outer portion of the top stretch 4b of the sleeve 4. The chamber C2 is in communication with the passage 23b of the filter and the passage 2d of the device 1.

As may be seen, by virtue of the structure described above, the device 1 can be arranged between a generic engine 30, having a lubrication circuit that comprises the conduit 32, the relief 33 and the holes 34, and a filter 20 that is suitable for that particular engine. This means that the male joint of the device 1, constituted by the externally threaded portion 4b of the sleeve 4 (see Figure 2), is substantially similar to the male joint of the engine 30, constituted by the externally threaded conduit 32, whilst the female joint of the device 1, constituted by the internally threaded portion 4a of the sleeve 4 (see Figure 2), is substantially similar to the female joint of the filter 20, constituted by the respective internally threaded wall 23a, said male joints being the same as one another and complementary to said female joints, which are also the same as one another. It is clear that the aforesaid joints, here illustrated and described merely by way of non-limiting example as threaded joints, could be of any other type suitable for the purpose, or of a type currently available on the market, within the limits and meeting the requirements of the invention.

Once again in Figure 3, the small arrows represent schematically the flow followed by the oil or other fluid that is to be detected using the device 1 and to be filtered by the filter 20. The flow of lubricating oil is induced, with modalities in themselves known, to come out of the engine 30 through the holes 34 of the end wall 31 and then penetrate into the chamber C1. The oil then penetrates into the chamber C inside the device 1 through the holes 3d present in the lid 3 (see Figure 2). As the oil ascends the chamber C, it impinges upon the sensor means 6-8 and then comes out through the holes 2d present in the wall 2a of the body 2 of the device 1 (see Figure 2). In this way, the oil reaches the aforesaid chamber C2. From the chamber C2 the oil enters the filter 20, through the holes 23b present in the end element 23 of the filter itself, to ascend the latter and traverse the filter element 22. The oil thus purified reaches the central part of the filter 20, in which a channel is defined, through which the oil itself is directed towards the opening delimited by the cylindrical internal wall 23a of the end element 23 of the filter 20. At this point, the purified oil penetrates into the sleeve 4, and then returns into the engine 30 through the conduit 32 of the latter.

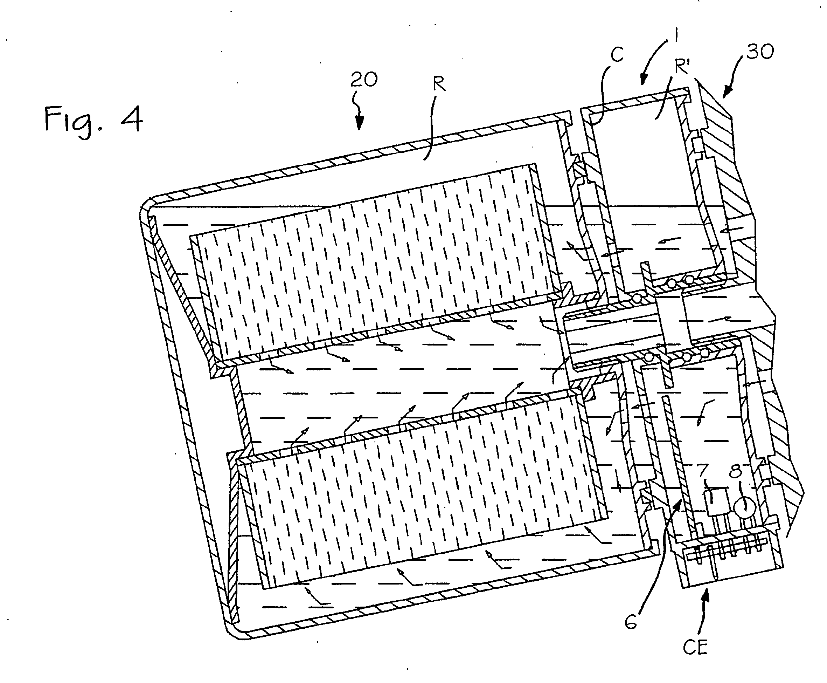

Represented in Figure 4 is a configuration of assembly of the device 1 different from that of Figure 3 in which, given the different conformation of the engine 30, the filter 20 and the detection device 1 are mounted in an inclined position (but said position could also be horizontal). In said figure the same references as those of Figure 3 are used. In the condition illustrated in Figure 4, within the filter 20 and the device 1 there may arise stagnation of air, in the areas designated, respectively, by R and R'. In the case where the device 1 were mounted with the sensor means 6-8 in a position corresponding to the area R', they could at least in part not be covered by the fluid present in the chamber C, with consequent errors of measurement or detection. In said anomalous operating conditions, i.e., with sensors or electrodes in part exposed and in contact with the air, there could moreover arise or be accentuated phenomena of oxidation and/or of corrosion of the sensor means 6-8 and/or of the detection electrodes themselve.

The electrodes of the sensor 6 can advantageously be shaped so as to occupy only a limited part of the chamber C, and in particular the part of chamber C close to the electrical connector CE, or in any case the part of chamber C that typically is constantly filled with the fluid or oil, such as in particular the part of chamber that is to be in the lower position. As has been said, the sleeve 4 is mounted so as to be able to rotate axially with respect to the casing formed by the body 2 and the lid 3 of the device 1. In this way, during assembly, the device 1 can be oriented angularly in the most appropriate way, for example, so that the sensor means 6-8 will be positioned in the lower position, as may be seen in Figure 4, i.e., in the part of chamber C that remains constantly filled with the fluid, even in the presence of stagnation of air in the area R'.

In such a perspective, a possible sequence of assembly of the device 1 could comprise at least some of the following steps:
- positioning of the device 1 on the engine 30, with the respective joint, i.e., the internally threaded portion 4a of the sleeve 4, fitted on the conduit 32 of the engine 30;
- insertion of a suitable tool, such as a spanner of an Allen type, in the respective seat (hexagonal, in the example) defined in the upper portion of conduit 4e of the sleeve 4 (see Figure 2);
- manual orientation of the device 1, so that the sensor means 6-8 will be located correctly, for example, as may be seen in Figure 4, i.e., in the lower part;
- rotation of the sleeve 4 using said tool, with consequent screwing of the internally threaded lower portion 4a of the sleeve 4 (see Figure 2) on the external thread of the conduit 32 of the engine 30, up to clamping of the device 1 in position and in a sealed way; and
- assembly of the filter 20 on the device 1, i.e., screwing of the female joint formed by the internally threaded central wall 23a of the filter 20 on the externally threaded upper portion 4b of the sleeve 4 (see Figure 2) up to clamping of the filter 20 in a sealed way on the device 1.

The temperature sensor 7, designed for detecting the temperature of the fluid, can be constituted by a negative-temperature-coefficient (NTC) resistor. The variable detected by the sensor 7, in addition to being of importance in absolute terms (i.e., as knowledge of the temperature of the fluid), can also be used for the purpose of "compensating" the measurements made by at least some of the other sensor means 6 and 8, this in view of the fact that some physical characteristics of a fluid can vary at its different temperatures (for example, the value of viscosity of the oil at a first temperature thereof is different from the one that can be detected at a different operating temperature of the same oil). In such a perspective, then, in the case where the quantities detected via the means 6 and 8 have to be compared with fixed-reference data or tables, the control logic that supervises operation of the device 1 will see to compensating said quantities detected in function of the temperature of the fluid existing at the moment of detection of the quantity of interest, as measured by the sensor 7. Alternatively, the control logic may envisage that given steps of detection will be made by the means 6 and 8 when the fluid reaches a pre-determined temperature, said point being detected by the sensor 7.

The generator of oscillations 8, constituted in the example by a quartz crystal, is used for the purposes of measurement of the viscosity of the fluid or of the oil, according to modalities in themselves known, and on the basis of the fact that the quartz crystal varies its own frequency of oscillation in function of the viscosity of the fluid with which it is in contact or in which it is immersed. Given that the viscosity depends to a certain extent upon the temperature of the fluid, the temperature sensor 7 is positioned as close as possible to the quartz oscillator 8 (and/or to the sensor 6) in order to obtain information as correct as possible as regards the viscosity and/or the characteristics of the fluid. It is to be noted that the electronic components referred to generically as "quartz components" usually comprise a casing for protection of a respective quartz crystal connected to two electrical contacts, which - when subjected to an appropriate voltage - oscillates at a given frequency. The component 8 is preferably without the aforesaid casing, and thus formed only by the quartz crystal, with respective connection terminals projecting into the hollow 2g. This moreover enables a further increase in the precision of measurement made using the quartz oscillator 8. Advantageously, there could be provided a perforated casing for the quartz crystal or for other sensor means, for example, with a shape similar to a mesh, said particular type of casing being designed to protect the quartz oscillator 8 or other sensors during any manipulation, for example, in the production stage and/or from accidental impact, albeit enabling the quartz crystal itself or other sensors to come into contact with the fluid to be detected. Said form of protection could advantageously be constituted by, or associated to, at least one component of the device, appropriately shaped and preferably made of thermoplastic material.

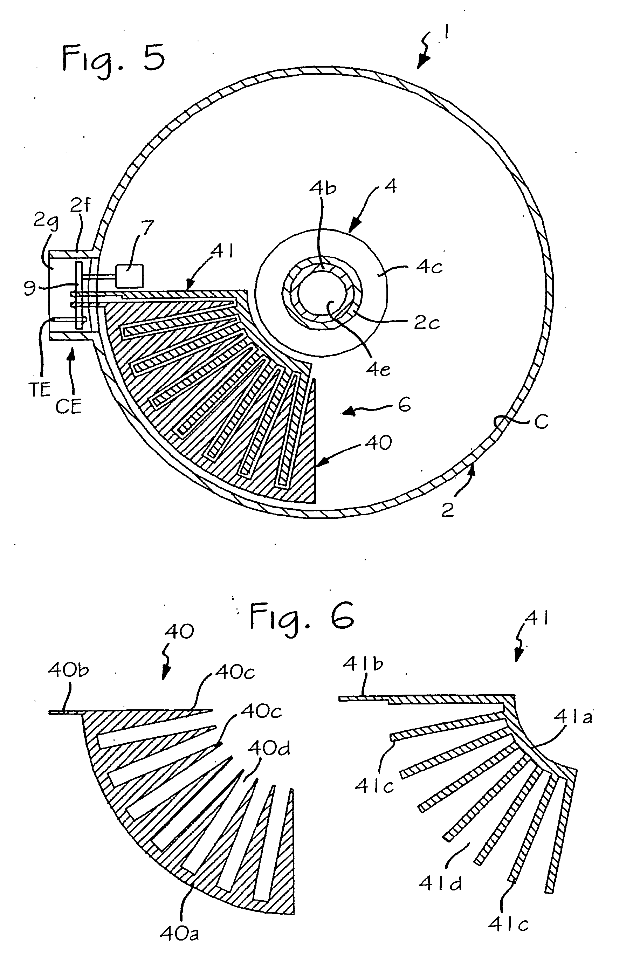

The sensor 6 is provided for detecting an electrical or physical quantity that can vary as a function of the quality of the fluid or of the oil, such as a value of resistance, resistivity, capacitance, acidity, etc. A first possible embodiment of the sensor 6 may be seen in the cross-sectional view of Figure 5, limitedly to its metal parts, constituted by two electrodes 40 and 41. In Figure 6 said electrodes 40 and 41 are visible individually, as obtained starting from at least a metal strap of reduced thickness, appropriately blanked and shaped. It should be noted that the sensor 6 preferably also has a frame or a support made of electrically insulating material, such as a thermoplastic material, not visible in the cross-sectional views of Figures 5 and 6, provided for supporting and holding in respective fixed positions the two electrodes 40 and 41, and/or for facilitating their assembly and/or housing in the casing of the device 1.

Each electrode 40, 41 comprises a respective base part 40a, 41a, substantially configured like the arc of a circle, from which there branches off a connection projection configured like a terminal 40b, 41b, in particular having a flattened shape. The terminals 40b, 41b preferably have a thickness equal or close to that of said strap from which the electrodes 40 and 41 have been obtained, or else equal to a multiple of said thickness, if the terminals are obtained by bending a portion of the strap back on itself. As may be noted, the development in length of the curved or arched base part 40a of the electrode 40 is greater than that of the homologous part 41a of the electrode 41 (in other words, the arc of a circle defined by the part 40a is longer than that defined by the part 41a). In the case exemplified, the at least partially curved or arched shape of the electrodes 40, 41 is advantageous in so far as it enables complete exploitation of the shape of circular section of the part of the body 2 in which the electrodes themselves are positioned. The terminal projections 40b, 41b are sealingly inserted in respective passages formed in the side wall 2b of the body 2, so as to project within the hollow 2g and here be connected to the connection support 9 (see what was described previously with reference to the terminals of the sensor means 6-8). In a variant (not represented), the terminals 40b and 41b and/or the terminals of the sensors 7 and/or 8 could directly form the terminals TE of the connector CE.

From the base part 40a of the electrode 40 there branch off laterally a series of first prolongations or teeth 40c having a substantially triangular or pointed shape, i.e., one that narrows towards the outer end, arranged so that they are spaced apart with respect to one another by spaces 40d. Said spaces 40d are delimited longitudinally by the facing surfaces of two successive teeth 40c. Said facing surfaces substantially coincide with the thickness of the strap in said area. Given the "arched" conformation of the electrode 40 and the shape of the teeth 40c, in the example illustrated in Figure 6 said facing surfaces are substantially parallel, even though other arrangements are possible (the facing surfaces could, for example, be convergent towards the outside or towards the inside, i.e., with spaces 40d that are narrower or wider, respectively, towards the pointed ends). In the embodiment illustrated in Figures 5 and 6, the teeth 40c and/or the corresponding pointed ends face the centre of the device 1, or of its body 2. From the base part 41a of the electrode 41 there branch off laterally a series of second prolongations or teeth 41c, having a substantially rectangular shape, arranged so that they are spaced apart with respect to one another by spaces 41d. Also said spaces 41d are delimited longitudinally by the facing surfaces of two successive teeth 41c. Said facing surfaces substantially coincide with the thickness of said strap in said area. In this case, given the "arched" conformation of the electrode and the shape of the teeth 41c, said facing surfaces are substantially divergent from one another. The teeth 41c could, however, have a different shape, for example, substantially pointed or triangular. Furthermore, the respective facing surfaces could be more or differently inclined with respect to one another, for example with spaces 41d still wider towards the outer part. In the example illustrated in Figures 5 and 6, the teeth 41c and/or the corresponding outer ends face the peripheral part of the device 1 or of its body 2 with respect to the tangent of which they are substantially orthogonal. As may be appreciated, the arc of a circle defined by the base part 40a of the electrode 40 has a development in length greater than an arc of an imaginary circle touched by the ends or tips of the respective teeth 40c. Instead, the arc of a circle defined by the base part 41a of the electrode 41 has a development in length smaller than an arc of an imaginary circle touched by the ends or tips of the respective teeth 41c. The conformation of the teeth 40c, 41c is such that, when the two electrodes 40, 41 are coplanar, in an operating position within the respective frame, the teeth 40c are intercalated with respect to the teeth 41c, without ever coming into contact therewith, as may be seen, for example, in Figure 5, with the facing surfaces of the teeth 40c and 41c substantially parallel to or equidistant from one another. The set of the two electrodes 40, 41 with interdigitated teeth has an overall shape of a circular sector with a development of approximately 90°.

As has been said, the electrodes 40 and 41 can be made each of a single piece from a strap, and hence in the form of a lamina having two parallel main faces or faces with larger area, between which said thickness of the lamina or strap is defined. In the case where the sensor 6 is of a capacitive type, the surfaces corresponding to said thickness, i.e., the perimetral surfaces obtained in the thickness of the metal strap of the two electrodes, form the plate surfaces (armatures) of the sensor 6. In a variant (not represented), on the other hand, the plate surfaces of the sensor 6 could be at least in part made up of two main faces or faces with larger area, of two respective electrodes, facing one another.

In the use of the sensor 6, when the electrodes 40, 41 are immersed in the oil and between them an appropriate voltage and/or current is applied and/or detected, possible variations of an electrical or physical quantity (conductivity, resistivity, capacitance, acidity, etc.) measured via the electrodes themselves can be evaluated electronically, in a known way, in order to have information regarding the level of degradation of the oil (in this regard, it should be considered, for example, that the conductivity of the oil increases with ageing thereof).

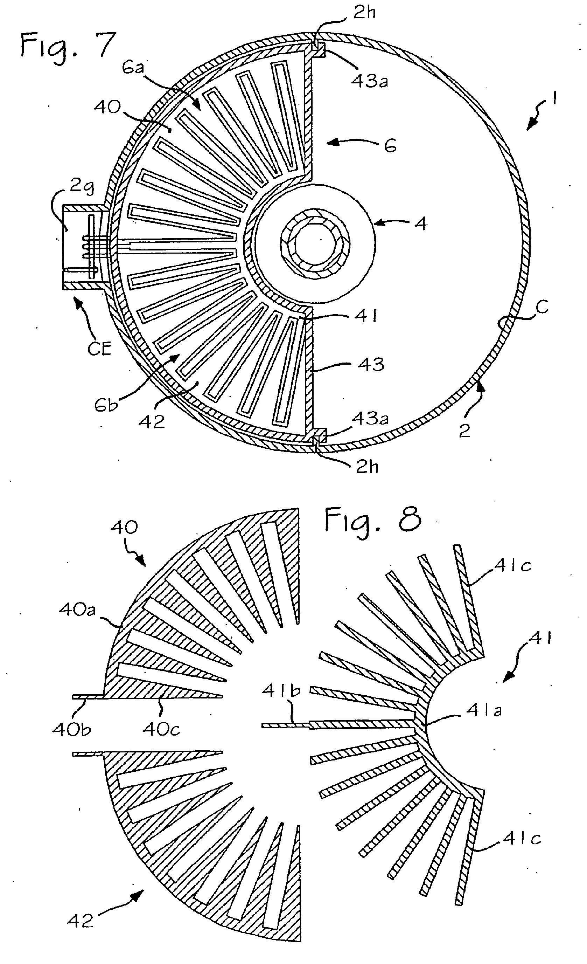

In Figures 7 and 8 a possible variant of the sensor 6 is represented, which comprises three electrodes 40, 41 and 42, the electrode 41 being common to the electrodes 40 and 42. Said three electrodes are held in fixed relative positions, preferably equidistant, without coming into contact with one another, by means of a respective frame made of insulating material, for example, overmoulded with thermoplastic material, here visible and designated by 43 in Figure 7. The frame 43 can comprise coupling elements 43a, designed to couple with respective coupling elements 2h made in the body 2. It is to be noted that in Figure 7 the frame 43 is visible in cross-sectional view, and the electrodes 40, 41 and 42 are directly in view, whilst in Figure 8 only the electrodes are visible in cross-sectional view. The electrodes 40 and 42 are built in a way substantially similar to one another and with respect to the electrode 40 illustrated in Figure 6. The electrode 41 is, instead, of conception similar to the electrode 41 illustrated in Figure 6, but with the respective base part 41a that extends according to an arc of a circle of approximately 180°. In this case, moreover, the flat terminal projection 41b of the electrode 41 branches off from one of its teeth 40c occupying an intermediate position, instead of from one end of the respective base part 41a. As may be appreciated, when the three electrodes 40, 41 and 42 are coplanar, in an operating position within the respective frame 43, the teeth of the common electrode 41 are intercalated with respect to the teeth of the two electrodes 40 and 42, without ever coming into contact therewith, as may be seen in Figure 7. The set of the three electrodes in this case forms the overall shape of a circular sector with a development of approximately 180°, and the sensor 6 comprises in effect two distinct sensors 6a, 6b, which can be controlled independently of one another, for example one for detecting a value of conductivity (or resistivity) and the other for detecting a value of capacitance.

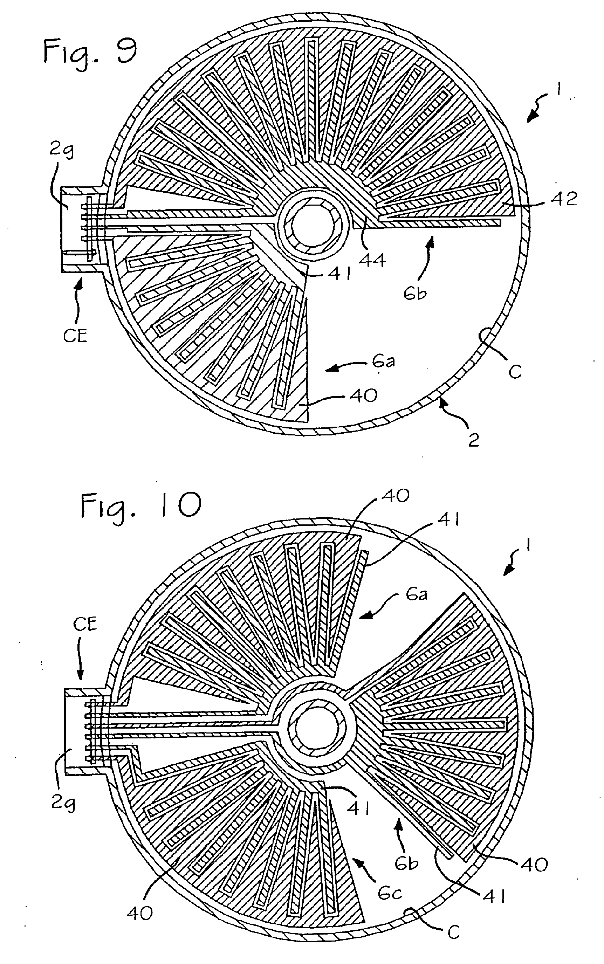

In Figure 9 a further possible variant embodiment of the sensor 6 is represented, in which two distinct pairs of electrodes, designated, respectively, by 40, 41 and 42, 44, are schematically represented in cross-sectional view, each pair forming a respective sensor 6a, 6b. Also said electrodes are preferably held in fixed relative position by a respective frame (not shown here). The electrodes 40 and 41 illustrated in Figure 9 are substantially configured like the corresponding electrodes illustrated in Figure 6. The electrodes 42 and 44 are of a conception similar to the electrodes 40 and 41, but with respective base parts that extend substantially on an arc of a circle of approximately 180°.

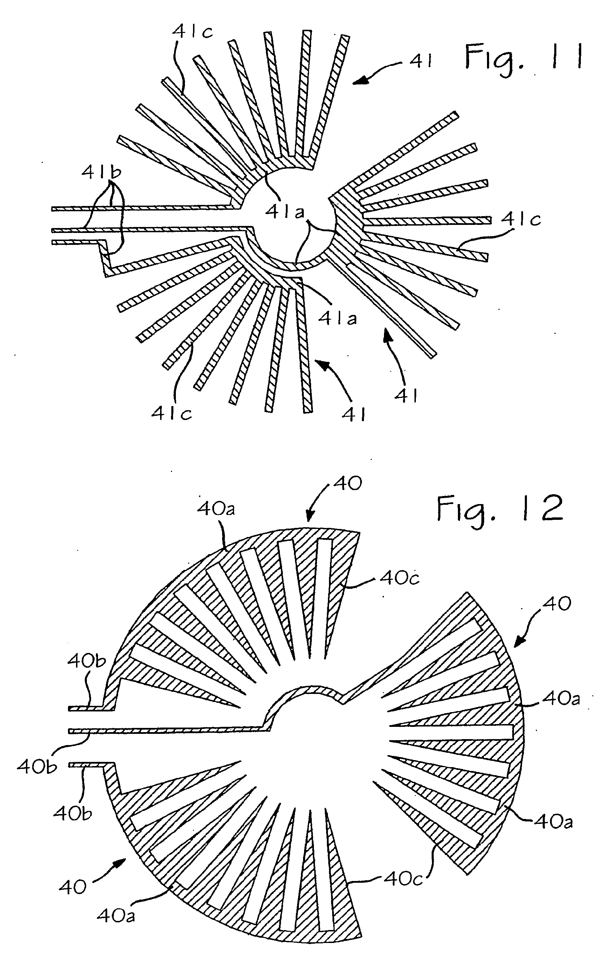

Figure 10 illustrates, instead, the case of use of three distinct pairs of electrodes, which form three distinct sensors 6a, 6b and 6c, evenly distributed and spaced within the chamber C, the various electrodes being preferably supported by a single frame (not visible). As may be seen in Figures 11 and 12, the first and the second electrodes of each pair, designated, respectively, by 40 and 41, are built in a way similar to that of the homologous electrodes illustrated in Figure 6, apart from the respective flat terminal projections 40b, 41b, appropriately shaped so as to reach inside the hollow 2g of the connector CE. In this variant, moreover, the base part 40a, 41a of the electrodes 40, 41 of each pair extends according to an arc of a circle of approximately 100°. In this case, the provision of three distinct pairs of electrodes evenly distributed and spaced within the chamber C enables availability of three distinct detection sensors 6a, 6b, 6c, so that at least one of them is certainly in contact with the oil, also in the presence of possible stagnation of air. The fact of having available three sensors that are substantially the same as one another moreover enables three distinct detections to be made, which can then be appropriately processed via the electronic control circuit of the device 1, such as, for example, making an average of the three values measured, or else a comparison between the three values measured, or else again an elimination or deviation of one of the three values detected, etc. Said arrangement likewise enables, if need be, verification also of possible faults, for example, as a result of a deterioration of one of the electrodes, or else due to deposits of foreign bodies on the electrodes themselves or between them, etc.

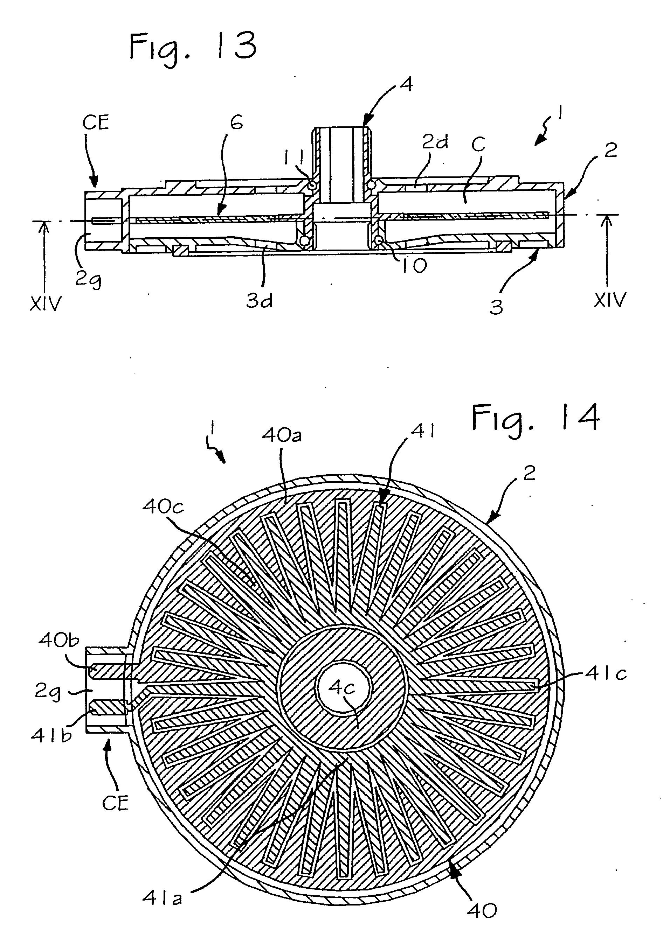

In Figures 13 and 14 a possible embodiment of the device 1 is represented, in which the sensor 6 is formed by a pair of electrodes 40, 41 built in a way conceptually similar to that of the homologous sensors illustrated in Figure 6, but which have a development that is circular as a whole. As may be noted with reference to said variant, the device 1 can also not envisage the further sensor means 7 and 8 and have a casing formed by the body 2 and by the lid 3 of limited height, so that it can be mounted between the filter 20 and the engine 30, without particular encumbrance, in the case where there exist, for example, constraints in height. It is to be noted in any case that, in general terms, the dimension of encumbrance in height of the device (understood as distance between wall 2a of the body 2 and wall 3a of the lid 3) is always decidedly smaller than its dimension of width (understood, in the case exemplified of a basically cylindrical casing, as diameter of the peripheral wall 2b of the body 2). As may be noted, the development in length of the circular base part 40a of the electrode 40 is greater than that of the homologous part 41a of the electrode 41 (in other words, the circumference defined by the part 40a is longer than the one defined by the part 41a). The circumference defined by the base part 40a of the electrode 40 is greater than an imaginary circumference touched by the ends or tips of the respective teeth 40c. The circumference defined by the base part 41a of the electrode 41 is, instead, smaller than an imaginary circumference touched by the ends or tips of the respective teeth 41c. In Figures 13 and 14, the terminals 40b, 41b of the electrodes are preferably shaped in such a way as to form at least part of the electrical connector CE, in particular a connector of the type designed for use in the automotive sector. Furthermore, in Figure 14 the different shape of the teeth 41c is visible, which present a profile that substantially narrows towards the outer end. Said shape enables a joint area to be obtained that is more ample and provides a greater strength than the base part 41a of the electrode 41, from which the teeth themselves branch off laterally.

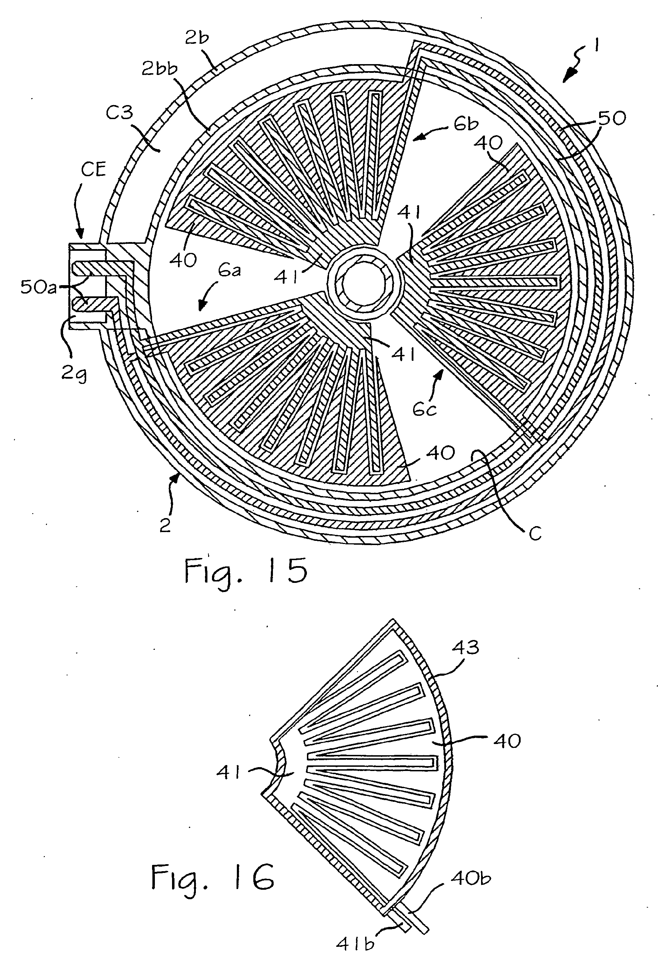

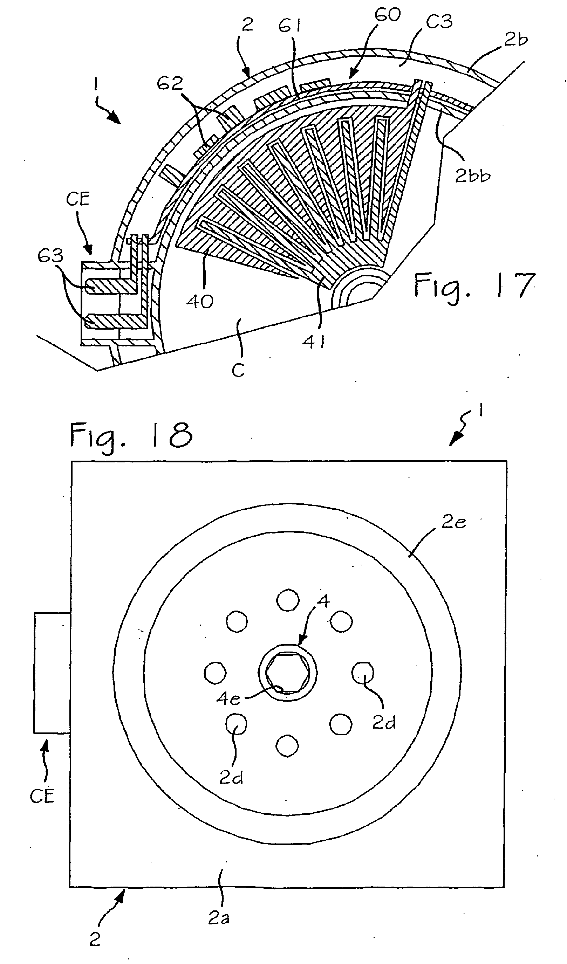

The further variant, built in a way referred to in Figures 15 and 16, is conceptually similar to that of Figure 10, envisaging, however, a different shape of the body 2 of the device 1, and different means of interconnection for the various electrodes 40, 41 of three pairs, i.e., of three sensors 6a, 6b and 6c. In particular, each pair of electrodes 40, 41 has a respective overmoulded frame 43, visible only in Figure 16, from which there comes out a respective flat terminal portion 40b, 41b, whilst the body 2 is configured so as to have an annular peripheral chamber C3, made between two walls 2b and 2bb of the body 2 and hermetically isolated from the chamber C of transit and detection of the fluid. Within the chamber C3 there are at least two connection elements 50, which each have a respective end or terminal 50a that projects within the hollow 2g of the connector CE, through respective passages that are appropriately sealed (for example, by means of resin treatment, overmoulding, etc.). The electrodes of each pair are mounted so that the ends of the flat terminal portions 40b, 41b that project from the respective frames 43 are positioned within the chamber C3, through respective sealed passages in the wall 2bb, to be connected there to the connection elements 50, with modalities in themselves known (welding, electrical spot-welding, etc.). The connection elements 50 could be, for example, in the form of insulated electrical wires or else of metal straps spaced at a distance from one another, blanked with appropriate geometries from sheet metal. In the chamber C3, which, if need be, could also have a geometry different from the one represented by way of example in Figure 15, there can also be housed an electronic circuit that supervises operation of the sensor means. In fact, in Figure 17, a possible variant in this sense is represented, where, located within the chamber C3, there is an electronic circuit 60, comprising at least one printed circuit 61, for example, of a flexible type, on which electronic components 62 are mounted, such as a microcontroller, electronic memory means, etc. In this case, the connection elements 50 and/or the terminal portions 40b, 41b are connected to said printed circuit 61, which in turn comprises terminals 63 for connection to the electrical connector CE.

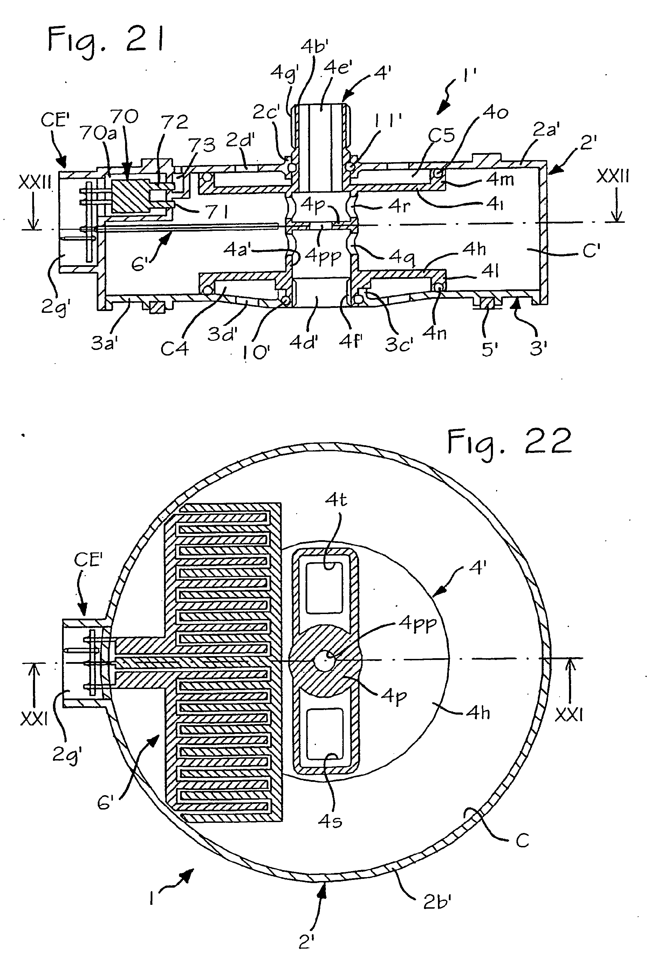

Figures 18 to 20 regard a possible variant embodiment of the sensor 6, provided in particular for the cases in which the body 2 of the device 1 does not have a circular shape, as may be seen schematically, for example, in Figure 18, and hence the electrodes are not necessarily arched. It is to be noted, in any case, that non-arched sensors can be used also in the case of a substantially circular body 2 (see, for example, the case of Figures 21-24).

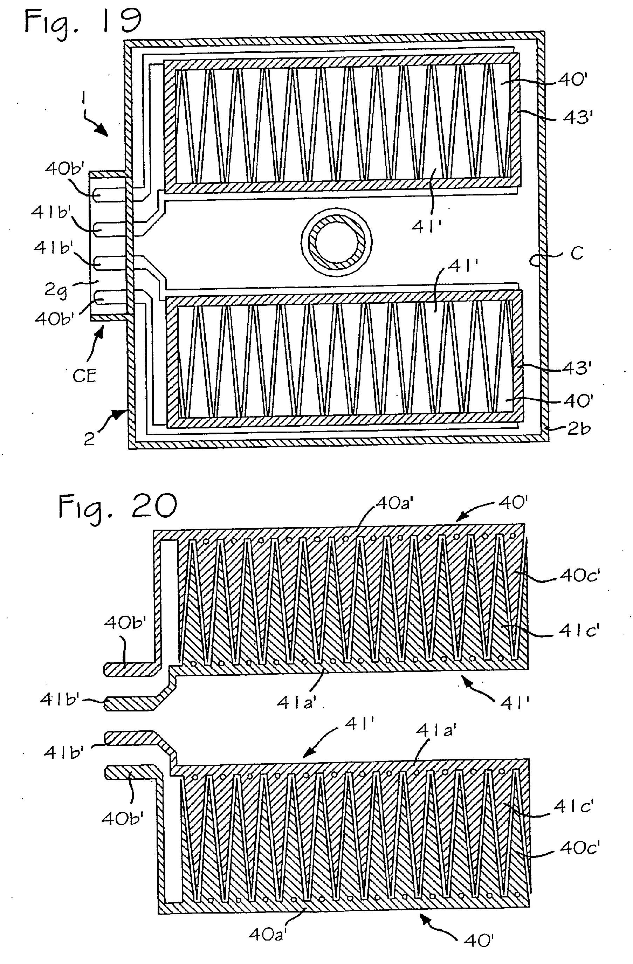

In the case of Figures 19 and 20, two series of electrodes 40' and 41' are provided, substantially similar to one another and in a specular position, obtained, for example, starting from at least one metal strap of reduced thickness, appropriately blanked and shaped. There is moreover provided a supporting frame 43' made of electrically insulating material, preferably a thermoplastic material, for supporting and keeping in fixed relative positions a respective pair of electrodes 40', 41'. As may be seen in Figure 20, each electrode 40', 41' comprises a respective base part 40a', 41a', that is substantially rectilinear and has a respective connection projection configured as terminal 40b', 41b', which is appropriately shaped. As may be appreciated in Figure 19, said projections 40b', 41b' are sealingly inserted in respective passages formed in the side wall 2b of the body 2, so as to project within the hollow 2g. From the base part 40a' of the electrode 40' there branch off laterally a series of first teeth 40c', having a substantially triangular or pointed shape, i.e., one which narrows towards the outer end, arranged so that they are spaced apart with respect to one another by spaces delimited longitudinally by the substantially divergent surfaces of two successive teeth 40c'. From the base part 41a' of the electrode 41' there branches off laterally a series of second teeth 41c', which also have a substantially triangular shape, arranged so that they are spaced apart with respect to one another by spaces delimited longitudinally by the substantially divergent surfaces of two successive teeth 41c'. Also in this case the conformation of the teeth 40c', 41c' is such that, when the two electrodes 40', 41' are coplanar, in an operating position within the respective frame 43', the teeth 40c' are intercalated with respect to the teeth 41c', without ever coming into contact therewith, as may be seen clearly in Figure 19, and with the respective facing surfaces substantially parallel. In a possible variant, three electrodes with a rectilinear base part can be arranged according to a configuration conceptually similar to the one represented in Figures 7-8, i.e., with two electrodes that are similar to one another and distinct from one another, and a third common electrode. An embodiment of this type is illustrated in Figures 21-24.

Obviously, the sensor means provided for detecting the electrical quantity or quantities of interest mentioned above could be built in a different way from the ones previously exemplified; for example, they could be of a type commonly available on the market.

Figures 21-26 regard a device according to the invention, in accordance with which the chamber where the sensor means operate, and in particular the sensor of electrical quantities, is to be in hydraulic connection with the outlet of the filter 20, i.e., an embodiment in which the detections are made by the device on the fluid already filtered. For the purposes of description of said figures, the same reference numbers as those of the preceding figures are used, indexed with the prime sign, as regards the device according to the invention, in order to designate elements that are equivalent to the ones already mentioned.

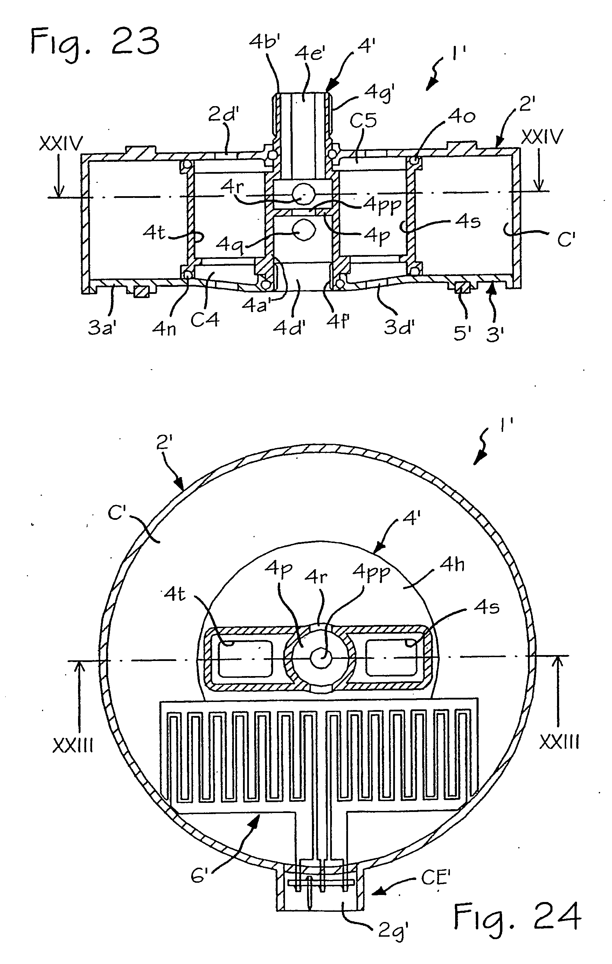

Also in said embodiment the device, designated by 1', comprises a casing formed by a body 2' and a lid 3', to which a sleeve 4' is coupled in a rotatable or angularly movable way. In the case of the variant in question, the body 2' and the lid 3' have a shape slightly different from the case of Figures 1-6, without, however, appreciable variations in their general structure, with the exception of the cylindrical central walls 2c' and 3c', which here have a development in height that is very limited. The body of the sleeve 4' comprises, instead, a flange-like lower portion 4h and a flange-like upper portion 4i, which are substantially circular, each comprising a respective cylindrical peripheral wall 41, 4m. In the assembled condition of the device 1, as may be seen for example in Figures 21 and 23, the aforesaid peripheral walls 41, 4m of the sleeve 4' rest, respectively, upon the internal surface of the walls 2a' and 3a' of the body 2' and of the lid 3', with interposed respective annular sealing means, for example, of circular cross section, such as the O-rings designated by 4n and 4o. In this way, the flange-like portion 4h of the sleeve delimits - together with the respective peripheral wall 41 and the respective sealing means 4n, the portion 4a' of the sleeve itself and the wall 3a' of the lid 3' - a chamber C4 isolated from the chamber C'. On the other side, the flange-like portion 4i of the sleeve delimits - together with the respective peripheral wall 4m and the respective sealing means 4o, the portion 4b' of the sleeve itself and the wall 2a' of the body 2' - a chamber C5, which is also isolated from the chamber C'. The aforesaid chambers C4 and C5 are built in a such a way (in particular having an annular or circular shape) as to be always in communication with the passages 3d' and 2d', irrespective of the angular position of the sleeve 4' with respect to the lid 3' and/or to the body 2' of the device 1. As compared to the embodiment illustrated in Figures 1-6, moreover, the lower portion 4d' and upper portion 4e' of the conduit inside the sleeve 4' are preferably separated from one another by means of an intermediate restriction 4p. In the proximity of said restriction 4p, the lower and upper portions 4a' and 4b' of the sleeve, which form its internal conduit 4d'-4e', are provided with respective side openings 4q and 4r, in communication with the chamber C'. The intermediate restriction 4p is preferably provided with a hole or central passage 4pp of calibrated cross section. Also the side openings 4q and 4r have calibrated cross sections, in particular for the purpose of obtaining a pre-defined sharing of the flow of the circulating fluid, i.e., of the fluid or oil leaving the filter 20. In a possible variant (not represented), the restriction 4p could be replaced by a closed wall, designed to separate the lower and upper portions 4a' and 4b', i.e., to interrupt the conduit 4d'-4e' inside the sleeve 4', preferably in an intermediate part thereof, and in particular for the purpose of deviating into the chamber C' the entire flow of the circulating fluid, i.e., of the fluid or oil leaving the filter 20.

The body of the sleeve 4 moreover defines two side conduits, designated by 4s and 4t in Figures 22-24, which are substantially parallel to the central conduit 4d'-4e' and set in communication the chambers C4 and C5. Said side conduits 4s and 4t, as well as the chambers C4 and C5, substantially create a passage for the oil that is to be filtered, without said contaminated oil being able to reach the chamber C' in which the sensor means 6' are located. It should in any case be noted that, also in the event of slight leakage from the sealing means 4n, 4o, 10', 11' no particular malfunctioning of the hydraulic circuit occurs in so far as the fluid or oil remains in any case confined, in particular, via the sealing means 5', 23d. The sensor means of the device 1 could be operative for detecting said anomalous condition.

As may be noted in Figures 22 and 24, the sensor means 6' comprise three electrodes substantially of the same types described previously, but here substantially configured according to a combination of some characteristics described with reference to the variants illustrated in Figures 7-8 and 19-20, i.e., with rectilinear teeth parallel to one another, one electrode being common to the other two electrodes so as to form two distinct sensors.

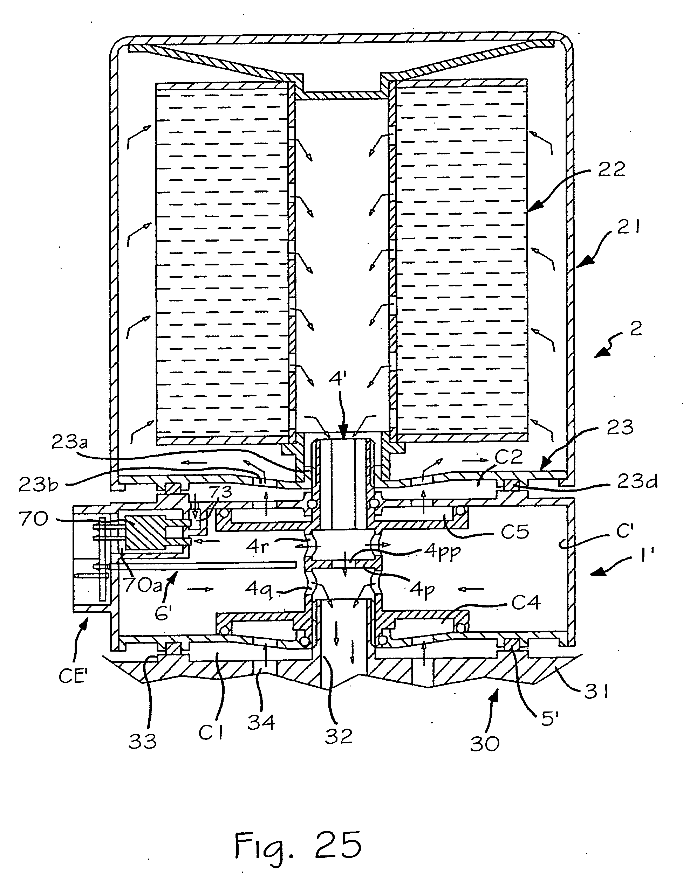

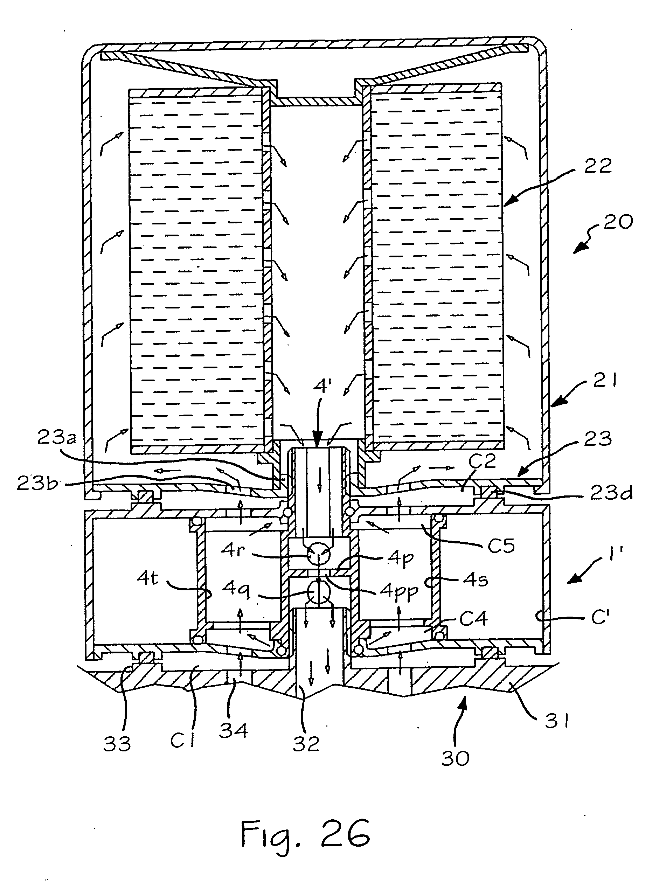

In Figures 25 and 26, the device 1' is illustrated in the working condition, i.e., arranged between an engine 30 and a filter 20. An installation of this sort is made with the modalities already described previously with reference to the embodiment illustrated in Figures 1-6. In said condition, the chamber C4 is in communication, via the holes 3d' of the lid 3' (see Figures 21 and 23), with the chamber C1, whilst the chamber C5 is in communication, via the holes 2d' of the body 2' (see Figures 21 and 23), with the chamber C2. Once again in Figures 25 and 26, the small arrows represent schematically the flow of the fluid or oil being detected via the device 1' and being filtered by the filter 20. The fluid is induced to flow out of the engine 30, via the holes 34 of the end wall 31, and then penetrate into the chamber C1, as in the previous embodiment. In this case, the oil then penetrates into the chamber C4, via the holes 3d' present in the lid 3' (see Figures 21 and 23), and then distributes in the chamber itself. The oil passes next into the conduits 4s and 4t, ascending them right to the top and then penetrating and being distributed in the chamber C5. The oil passes then into the chamber C2, through the holes 2d' of the wall 2a' of the body 2 (see Figures 21 and 23), and then enters the filter 20, via the holes 23b present in the end element 23 of the filter itself. The oil ascends the filter 20 and then traverses the filter element 22. The oil thus purified reaches the central part of the filter 20, in which there is defined the channel through which the oil itself is directed towards the opening delimited by the cylindrical internal wall 23a of the end element 23 of the filter 20. At this point, the purified oil penetrates into the portion 4b' of the sleeve 4 (see Figures 21 and 23). As a consequence of the presence of the restriction 4p, a part of the oil is induced to penetrate into the chamber C', through the holes 4r, filling said chamber and thus impinging upon the sensor means 6'. A residual part of oil passes, instead, through the passage 4pp of the restriction 4p, reaching the portion 4a' of the sleeve 4' (see Figures 21 and 23). The oil that has penetrated into the chamber C' comes out of it via the openings 4q, so that also this oil reaches the portion 4a' of the sleeve 4' (see Figures 23 and 24). From the portion 4a' of the sleeve 4' the oil can then return into the engine 30, through the conduit 32 of the latter. The restriction 4p, with the respective passage 4pp, is in particular designed to determine an increase in pressure in the upper portion 4e' of the conduit inside the sleeve 4', i.e., upstream of the restriction, and to determine a reduction of pressure in the lower portion 4d' of the conduit inside the sleeve 4', i.e., downstream of the restriction, according to known phenomena (for example, the Venturi effect). In this way, a circulation of the fluid in the chamber C' is induced albeit without having necessarily to close completely the central passage of the sleeve 4'. The calibration of the openings or passages 4r, 4q and 4pp, enables definition of the flow rate of the portion of fluid circulating in the chamber C', in particular for the purpose of obtaining a continuous change of the fluid being detected, albeit without disturbing said fluid excessively, and hence enabling a more precise measurement to be obtained.

As may be appreciated then, in the variant referred to in Figures 21-26, the device 1' sees to making the respective detection or detections on the oil or other fluid that has already been subjected to filtering, instead of on the contaminated oil, as, instead, occurred in the case of the embodiment illustrated in Figures 1-6. Said variant enables use of sensors 6-8 of relatively large dimensions, of the type that could not, instead, be inserted in the small space available in the central conduit for exit of the filtered fluid. In the variant illustrated in Figures 21-26, the device 1 is also equipped with a pressure sensor 70 in itself known, located in a respective housing chamber 70a formed in the body 2'. In the case exemplified, the pressure sensor 70 is of a differential type and is provided for said purpose with a first inlet 71, for detecting a first pressure, and with a second inlet 72, for detecting a second pressure, where in particular the first inlet 71 is in direct communication with the chamber C', i.e., with the oil leaving the filter 20, whilst the second inlet 72 is in communication with a further chamber 73 (see Figure 21), formed in the body 2 and connected hydraulically to the chamber C2, i.e., in communication with the oil that is to be introduced into the filter 20 (see also Figure 25). The pressure sensor 70 can be mounted directly on the printed circuit 9, with the respective housing chamber 70a, which, even though it is hermetic with respect to the chamber C' on account of appropriate seals, comprises hydraulic connection means 71, 72 for the sensor 70. The housing chamber 70a formed in the body 2' for housing the pressure sensor 70 is in a position corresponding to, and in communication with, the electrical connector CE' and is provided with appropriate passages for the first and second inlets 71, 72 of the sensor itself, said passages being preferably provided with appropriate sealing means (not represented) for isolating the chamber 70a with respect to the chamber C'. Said sealing means can, for example, be formed by elements made of elastic material, such as gaskets, O-rings, resins, or by means of any other known element suitable for the purpose. The pressure sensor 70 is connected electrically to the respective printed or electronic circuit, for example, of the type similar to the ones already mentioned with reference to the preceding examples, possibly combined or integrated in the device 1', said circuit being designed to control the sensor itself for the purposes of detection of the pressures at the inlets 71 and 72, verifying or calculating the corresponding pressure variations. Via the sensor 70 it is, for example, possible to monitor the pressure of the fluid leaving the device 1', i.e., entering the filter 20, and/or the pressure of the fluid entering the device 1', i.e., leaving the filter 20, and detecting possible significant differences. The aforesaid electronic circuit, which preferably comprises a microcontroller or a microprocessor and appropriate memory means, is able to compare the values of pressure measured with pre-defined data contained in said memory, in particular for the purpose of detecting a possible clogging of the filter 20.

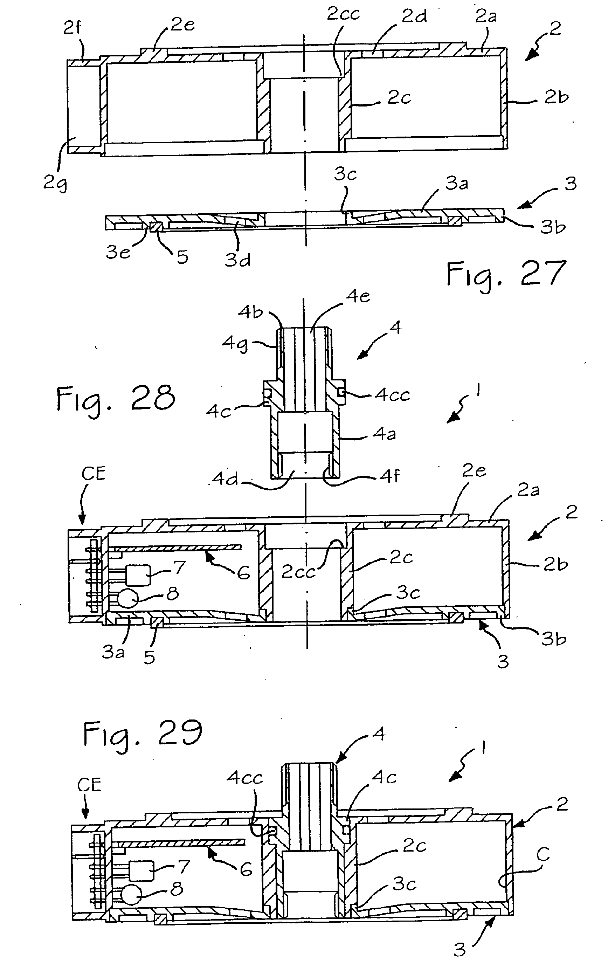

In Figures 27-29 and 30-33 two further possible variant embodiments are represented, the basic structures of which are substantially similar to the ones of the devices illustrated in Figures 1-6 and 21-26, respectively, but characterized, however, by a configuration of a modular or adaptable type. On this point, it should be considered that, albeit all having a substantially normalized or standard structure, different types of cartridge oil filters are currently available on the market, which substantially are differentiated from one another in dimensional terms, understood as height and external diameter of the filter body, and as regards the way in which the respective hydraulic/mechanical attachment, understood as diameter of the gasket 23d, type or diameter of the threaded joint 23a, thread pitch, etc., is made. For the purposes of the invention, of particular interest are precisely said different types of threaded joint 23a and of sealing gasket 23d of the filter 20, in relation to the joint of the device, constituted by the upper portion of the sleeve, and to the gasket of the device itself. Considering for example, as regards the filter, about ten different diameters of the gasket 23d and about ten different types of threaded joint 23b, there clearly emerges the large number of possible combinations between these, with a corresponding number of different possible types of filters. If it were necessary to envisage devices according to the invention that can couple with said variety of filters, it would become necessary to provide as many different devices according to the invention.

The variants referred to in Figures 27-29 and 30-33 are precisely aimed at limiting said drawback, through a modular embodiment of the detection device, where the connection sleeve can be substantially separated from the casing or body of the device and/or replaced. In this way it is possible to produce, for example, about ten different types of basic casing for the detection device, each provided with an appropriate sealing gasket, and to produce separately about ten different types of sleeves, each provided with an appropriate threaded attachment. Consequently, once again by way of non-limiting example, with about twenty components it is, for example, possible to produce about a hundred different variants of the device. More specifically, about ten casings and about twenty sleeves, compatible with the types of filters currently available on the market, enable, for example, production of some hundreds of variants of the device 1. In such a perspective, the components that can be produced in the greatest number of variants can be the sleeve, which is preferably formed of a single piece (obtained, for example, by moulding or injection-moulding material, such as a thermoplastic material or a metal in a die), and hence can be produced at a relatively low cost.

The individual components of the device can possibly be sold separately, enabling the end customer or end user (for example an installer), to purchase and/or make the combination of elements that are required at a given moment. Said solution enables a smaller number of finished devices to be kept in the warehouse, with consequent lower running costs, smaller occupation of space, smaller number of product codes to be handled, etc.

The device 1 according to the variant illustrated in Figures 27-29 is, in general terms, similar to the device illustrated in Figures 1-6, with the substantial difference that the cylindrical central wall 2c of the body 2 has a greater development in length, and the cylindrical central wall 3c of the lid 3 has a smaller development in length, as may be seen, for example, in Figure 27. The ends of said two walls 2c, 3c are moreover shaped in such a way as to be coupleable directly to one another, as may be seen in Figure 28, following upon the positioning of the lid 3 with respect to the body 2, where the points of union between the parts are hermetically sealed, for example, using glues, resins, etc. and/or welded to one another, for example, via laser re-melting of material, heat welding, etc. Alternatively, a mutual fixing of the female-thread/male-thread type could be envisaged, or a fast coupling such as a press-block coupling, a bayonet coupling, or a snap-action coupling, for example, with fixing parts, respectively, made in the body 2 and in the lid 3. In a variant (not represented), the wall 3c could be omitted, with the wall 2c that operates directly in a sealed way with respect to the end wall 3a of the lid 3. In a further variant (not represented), the wall 2c is instead omitted, and the wall 3c extends up to the end wall 2a of the body 2, where it is sealed. Also in said variants, the various parts 2, 2a, 2c, 3, 3a, 3c envisaged are joined with the modalities described previously.

To return to the example provided, the wall 2c moreover defines a housing, which defines a respective abutment surface, designated by 2cc in Figures 27 and 28, and which is designed to receive a respective portion projecting from the body of the sleeve 4, designated by 4c in Figure 28, said portion 4c being equipped with at least one respective peripheral sealing element 4cc. In this way, in the positioning seat formed by the walls 2c, 3c the sleeve 4 can be engaged in a sealed way. The abutment surface 2cc could also co-operate with the portion 4c of the sleeve 4 in order to clamp the device 1 in position. For said purpose, the abutment surface 2cc (but also another part of the seat of the sleeve) and the sleeve 4 can comprise means of mutual engagement, for example, in the form of reliefs or areas of knurling, designed to co-operate with one another to ensure correct positioning of the device 1 after this has been fixed in position, said fixing being obtained in particular by means of screwing of the sleeve 4 on the engine, with the body 2, 3 set between.

As may be appreciated, according to the variant proposed, various types of sleeves 4 may be associated to one and the same casing of the device 1, preferably formed by the body 2 and by the lid 3, said sleeves 4 being differentiated from one another in terms of dimensions of the respective internal threads 4f and external threads 4g. In the same way, one and the same sleeve 4 may be associated to various types of casings, for example, differentiated from one another in terms of diameter/dimensions of the reliefs 2e, 3e and of the gasket 5.

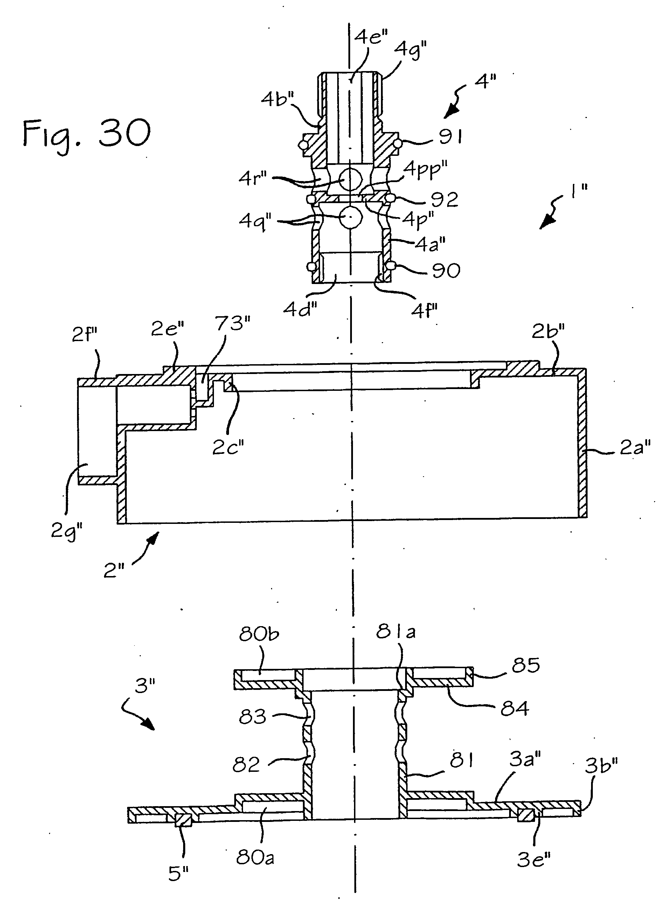

In the case of the embodiment of the invention illustrated in Figures 30-33 (in which the same reference numbers used in the preceding figures are again used, with the addition of the second-prime index for the ones regarding the device according to the invention), the device 1" is, in general terms, of conception similar to the device illustrated in Figures 21-26. However, there are substantial differences; namely, in this case:
- the central sleeve 4" is configured for being removable from the casing of the device 1", in order to enable its replacement and/or rotation and/or screwing and/or slotting into position, so as to allow the type of attachment and/or enable a proper orientation of the device;
- the body 2" is shaped for coupling, at its two axial ends, to the lid 3" in a sealed way;
- the lid 3" is shaped so as to:
- provide entirely a positioning seat for the sleeve 4";
- integrate the conduits 4s" and 4t";
- integrate at least part of the passages 4r and 4q of the preceding embodiment;
- possibly integrate the chambers C4 and C5 of the preceding embodiment.

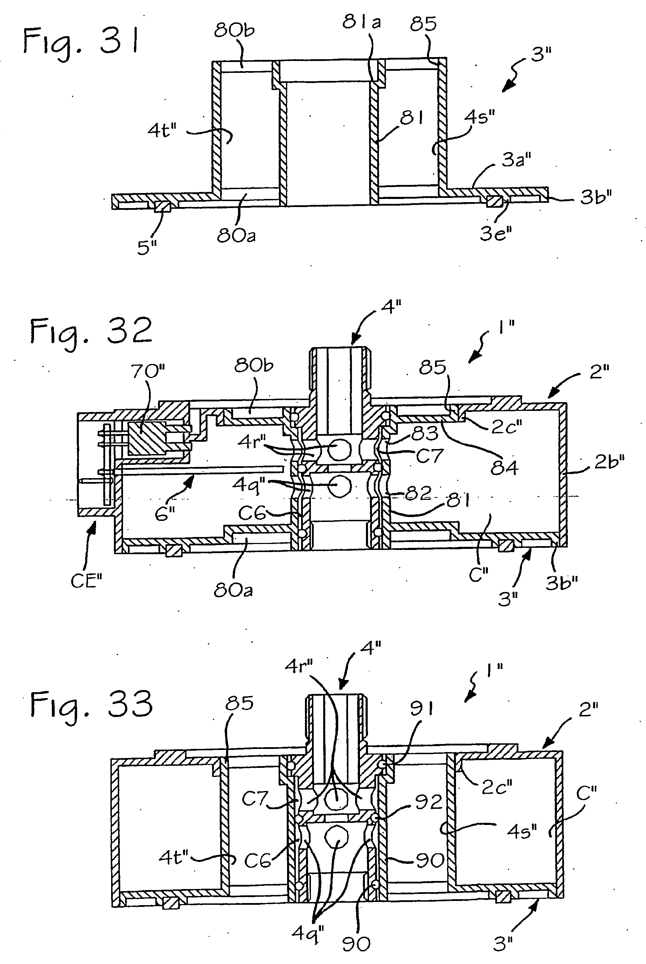

In particular, as may be seen in Figures 30 and 31, the wall 3a" of the lid 3" has a central seat or depression 80a, traversed by a tubular positioning wall 81, which proceeds further upwards, where it is equipped with two series of holes, designated by 82 and 83, respectively. Defined inside the tubular wall 81, in the upper part of the latter, is an abutment housing, designated by 81a. Once again, in the proximity of the upper end of the tubular wall 81, but on the outside thereof, a second flange-like portion 84 is provided, from which there rises a perpendicular, preferably cylindrical, peripheral wall 85. Said flange-like portion 84 and peripheral wall 85 are designed to form a seat or depression 80b, similar to the depression 80a, but with opposite orientation and/or arrangement. As may be seen in particular in Figure 31, in which the lid 3" alone is illustrated, in a cross-sectional view rotated through 90° with respect to that of Figure 30, there extend between the depression 80a and the depression 80b the conduits 4s" and 4t", parallel to the tubular wall 81. In the upper wall 2a" of the body 2" there is always present a central opening, delimited by an internal cylindrical wall 2c", which, however, in this case has a diameter decidedly larger than that of the embodiment illustrated in Figures 21-26. Said internal wall 2c" is to house and/or be coupled in a sealed way with the wall 85 of the top flange-like portion 84 of the tubular wall 81.

The sleeve 4" has a substantially tubular configuration, having a respective lower part 4a" and upper part 4b", each defining within it a respective portion of conduit 4d", 4e". Inside the part 4a" a respective female thread 4f" is formed, whilst outside the part 4b" a male thread 4g" is formed. Also in this case, the lower portion 4d" and upper portion 4e" of the conduit inside the sleeve 4" are preferably separated from one another by means of an intermediate restriction 4p" having a respective passage 4pp" (or else via a completely closed wall, according to a variant not represented). In the proximity of said restriction 4p", the lower and upper portions 4a" and 4b" of the sleeve are provided with respective side openings 4q" and 4r". Various peripheral sealing elements are associated to the body of the sleeve 4", and precisely at least one lower seal ring 90, one upper seal ring 91 and one intermediate seal ring 92, the latter being set substantially in a position corresponding to the restriction 4p", i.e., between the side openings 4r" and 4q".

As may be seen in Figures 32 and 33, for the purposes of fabrication of the device 1", the lid 3" is inserted from beneath in the body 2", to which the various sensor means, herein constituted by an electrode sensor 6" and a pressure sensor 70", together with the corresponding electrical connection means, have previously been associated. The internal wall 2c" of the body 2 is coupled in a sealed way, for example, via a sealant or by welding or screwing, etc., with the wall 85 of the upper flange-like portion 84 of the tubular wall 81 of the lid 3". In the same way, the peripheral wall 3b" of the lid 3" is coupled in a sealed way to the peripheral wall 2b" of the body 2". The body 2" and the lid 3" can possibly be provided with sealing elements and/or engagement elements (not represented) in order to obtain a better mutual seal and/or mechanical coupling. Said sealing elements and/or engagement elements can, for example, be of the press-block coupling type, in particular to speed up the operations of assembly and thus reduce the production costs. It should be noted that in the aforesaid configuration of assembly, the areas of coupling between the body 2" and the lid 3" are located on two superimposed faces of the device 1", preferably on the two surfaces that house the threads or coupling means 4f" and 4g".

At this point the sleeve 4" can be inserted within the tubular wall 81 formed in the lid 3". It will be appreciated in this regard that the diameter of the tubular wall 81 and of the corresponding abutment housing 81a illustrated in Figure 30 is slightly greater than that of the respective portions of the sleeve 4". In this way, following upon the aforesaid insertion, the lower seal ring 90 operates in a sealed way in the lower area of the tubular wall 81, the top seal ring 91 operates within the housing 81a, and the intermediate seal ring 92 operates in a sealed way in the area of the tubular wall 81 intermediate between the holes 82 and 83. In this way, the holes 4s" and 4r" of the sleeve 4" emerge in respective annular chambers C6 and C7 (see Figures 32 and 33), where in particular:
- the chamber C6 is delimited by the rings 90 and 91, as well as by the respective stretches of the tubular wall 81 and of the sleeve 4" that extend between said rings;
- the chamber C7 is delimited by the rings 91 and 92, as well as by the respective stretches of the tubular wall 81 and of the sleeve 4" that extend between said rings.

The provision of the chambers C6 and C7 enables screwing, rotation, or angular orientation of the device 1" with the modalities described previously with reference to the embodiment illustrated in Figures 1-6, without rendering necessary the alignment of the holes 4s" and 4r" of the sleeve 4" to the holes 82 and 83 of the tubular wall 81. In this way a passage is guaranteed for flow of fluid circulating in the device and/or in the filter, independently of the respective angular positions of the sleeve 4" with respect to the lid 3" or to the body 2".

As may be appreciated, operation of the detection device 6" is substantially similar to what has already been described in relation to Figures 21-26, with the difference that the purified oil leaving the filter 20 reaches the chamber C' via the holes 4r" of the sleeve 4", the chamber C7, and the holes 83 of the tubular wall 81, whilst the oil leaving the chamber C' returns into the sleeve through the holes 82 of the tubular wall 81, the chamber C6, and the holes 4q" of the sleeve 4".

Irrespective of the embodiments referred to above, the control circuit of the device according to the invention, whether this is integrated or otherwise in the device itself, preferably comprises at least one measurement circuit, in particular for generating and/or detecting electrical signals, for example, comprising at least one amplification circuit, preferably of a programmable type at least as regards the corresponding gain, and/or an automatic calibration circuit, preferably of the type designed to vary a digital datum or the value of a component, such as, for example, a resistance and/or one or more electronic switches or control devices. The control circuit preferably comprises also at least one digital processor, such as a microprocessor or a microcontroller, equipped with, or combined to, electronic memory means, preferably of a non-volatile type and/or of an electronically rewritable type, for example, of the EEPROM or Flash type. Said memory means are pre-arranged for storing information in a permanent way even in the absence of electrical supply and/or are designed to be readable and writable also after being mounted or assembled on the respective electronic circuit. Said memory means stores at least part of the control logic and/or of the data necessary for operation of the device. The aforesaid microcontroller can be supplied through a respective supply stage, of a type in itself known, which can vary the output voltage according to the requirements of the circuit. Preferably a line for transmission and/or reception of signals (in the form of logic states 1 and 0, and/or analog data, and/or voltage values, etc.) is associated to the aforesaid circuit, for example, of a serial type with standard protocol. Said line is preferably used both for the purposes of programming the microcontroller and for transmitting therefrom information regarding operation of the circuit and its dialogue with other systems on-board the vehicle (engine control unit, diagnostic systems, etc.).

The aforesaid line can moreover be exploited in the stage of testing of the device. In such a perspective, data detected in the testing stage via the sensor means of the device can be compared with data detected via an external measuring instrument, such as an oscilloscope or other system of measurement and testing in the production cycle. For said purpose, during at least the production stage there can advantageously be provided the electrical and/or hydraulic connection of the device according to the invention to an external testing apparatus. The device can be connected electrically to the aforesaid electronic apparatus, for example, via the connector CE', CE", and connected hydraulically via at least one of its conduits for transit of the fluid to the testing apparatus, which will be designed to supply a sample of fluid, in order to have available at least one reference parameter for testing. A possible stage of testing and/or calibration of the device could thus envisage at least steps such as:
- detection or measurement of at least one characteristic of the aforesaid fluid sample via the device 1', 1";
- transmission of the value measured by the device 1', 1" to the electronic testing system;
- verification or comparison of the value measured with a respective standard or reference value;
- processing, according to the aforesaid comparison, of a calibration value, such as, for example, a value of the gain of an amplifier circuit of the device 1', 1";
- sending of said calibration value from the electronic testing system to the electronic circuit of the device 1', 1", or, programming of said circuit.

The aforesaid steps can, if need be, be repeated a number of times, until the desired measurement value is reached, for example, by varying each time the aforesaid calibration value.

A possible variant can, instead, envisage processing of the values detected directly in the electronic circuit of the device 1', 1". In this case, the stage of testing and/or calibration of the device could thus envisage at least one of the following steps:
- detection or measurement of a characteristic of the fluid sample via the device 1', 1";
- transmission of the reference or standard value from the electronic testing system to the electronic circuit of the device 1', 1";
- verification or comparison of the value measured with the reference value;
- processing, according to the aforesaid comparison, of a calibration value, for example, the value of the gain of an amplifier circuit of the device 1', 1".

Also in this variant of the testing and/or calibration cycle, the aforesaid steps can be repeated a number of times until the desired measurement value is reached, finally transmitting an appropriate signal to the automatic control system.

Automatic calibration could, for example, envisage writing or recording of at least one datum or value in a memory, preferably of a non-volatile type and/or variation of the value of a component, for example, the value of a digital potentiometer, the resistance of which could, for example, be used to define the gain of an amplifier circuit, connected to which there is at least one of the detecting means of the device according to the invention.

The device according to the invention is economically advantageous to produce given that its components number relatively few, and some of them are commercially available (for example, the sensor means), whilst others can be readily obtained at a low cost. Also the assembly of the device is convenient and fast, by virtue of the geometries of the components, which are preferably formed by a small number of items.

The basic items of the structure of the device (body 2', 2", lid 3', 3", sleeve 4', 4") are preferably made of moulded thermoplastic material, and hence obtainable in a simple and economically advantageous way. There is nothing to rule out, in any case, making the parts of the casing of the device of thermoplastic material and the sleeve of metal material, or a material of some other type, in particular for the purpose of obtaining an electrically insulated casing and a very sturdy sleeve, in order to enable forceful screwing thereof. In the same way, at least part of the body 2', 2" and/or of the lid 3', 3" could be made of metal material, or else with a series or combination of a number of materials.

Also the sensor means for detecting electrical quantities are obtainable via processes that are in themselves elementary, such as simple operations of blanking for the formation of the electrodes, and simple operations of overmoulding for the formation of the respective frames and/or of the body of the device itself.

It should be emphasized how, according to a further aspect of the invention, the electrodes of the sensor means that can be used in a device for detecting the characteristics of a fluid, such as in particular a lubricating oil, can be formed at least in part with a plastic material or a synthetic material that is electrically conductive or rendered such, for example, via operations of moulding or injection-moulding. Said electrodes can be made as independent components and then be mounted on the respective device, or else can be directly overmoulded on, or co-moulded with, at least part of the body of the device itself, or one or more of its components. Said moulding operations can, for example, be performed using systems of injection-moulding and/or thermoforming and/or vulcanization of materials, such as thermoplastic and/or thermosetting materials and/or elastomers and/or other equivalent materials suitable for the purpose.

In said solution, the electrodes can be made of substantially insulating thermoplastic material comprising, or charged with, an electrically conductive material or substance, such as, for example, carbon fibres or powder, graphite, metallic material, conductive synthetic substances, etc. For said purpose it is particularly advantageous to use a material with carbon-fibre fillers, which is more resistant to chemical and/or electrochemical aggression of the substances in which the electrodes are immersed. In combination, or as an alternative, the synthetic material used for forming the electrodes could be of an intrinsically conductive type from the electrical standpoint by virtue of its molecular structure, without the need for other additional materials or substances being used as fillers or additives; this is, for example, the behaviour of certain so-called "inherently conductive" polymers. Where not otherwise specified, and in the annexed claims, by the term "electrically conductive synthetic material" is to be understood indifferently a synthetic material to which there is added another electrically conductive material or substance, or else a synthetic material intrinsically conductive from the electrical standpoint. Furthermore, the aforesaid synthetic material can also be in the form of electrically conductive ink, appropriately distributed or silk-screen printed in appropriate areas of the device, or else on one or more of its components or parts thereof.

The electrically conductive synthetic material could also form at least part of a respective external electrical connector, such as its elements of electrical connection. Alternatively, said conductive moulded synthetic material could be electrically connected to metallic terminals of the connector, for example, overmoulded on a part or an end of the latter.

In a further variant, the electrodes can envisage an electrically conductive core, subsequently coated or overmoulded with electrically conductive synthetic material, which extends for a substantial part of the length of the electrode. Said core can be made, for example, of metal material or else using a carbon-fibre thread having relatively low electrical resistance, made up of a multitude of wires or capillary fibres. Electrically conductive thermoplastic material is then overmoulded on said core to form the finished electrodes, as distinct elements, which are then assembled on the device, or else as elements directly overmoulded on the device. The material of said core has in particular a lower electrical resistance, or a higher electrical conductivity, as compared to the analogous parameter of said electrically conductive synthetic material.

Consequently, with reference to the device 1', 1" described previously, one or more of the electrodes 40, 41, 42, 44 of the sensor 6, 6', 6" in the various configurations can be formed, instead of by blanked metal strap, via moulding of thermoplastic material, either comprising or with the addition of an aforesaid electrically conductive material, as explained above. In the case where the electrodes 40, 41, 42, 44 are equipped with a supporting frame made of insulating material (for example, of the same type as the ones designated by 43 in Figures 7 and 16 and by 43' in Figures 19 and 20), this can be co-moulded with the electrodes made of conductive plastic or overmoulded on them. Another possibility is that of co-moulding the electrodes with said frame or overmoulding them on said frame. Said frame could be formed by a part 2'-4', 2"-4" of the body of the device 1', 1" or be made during moulding of said part, possibly co-moulded with or overmoulded on the electrodes.

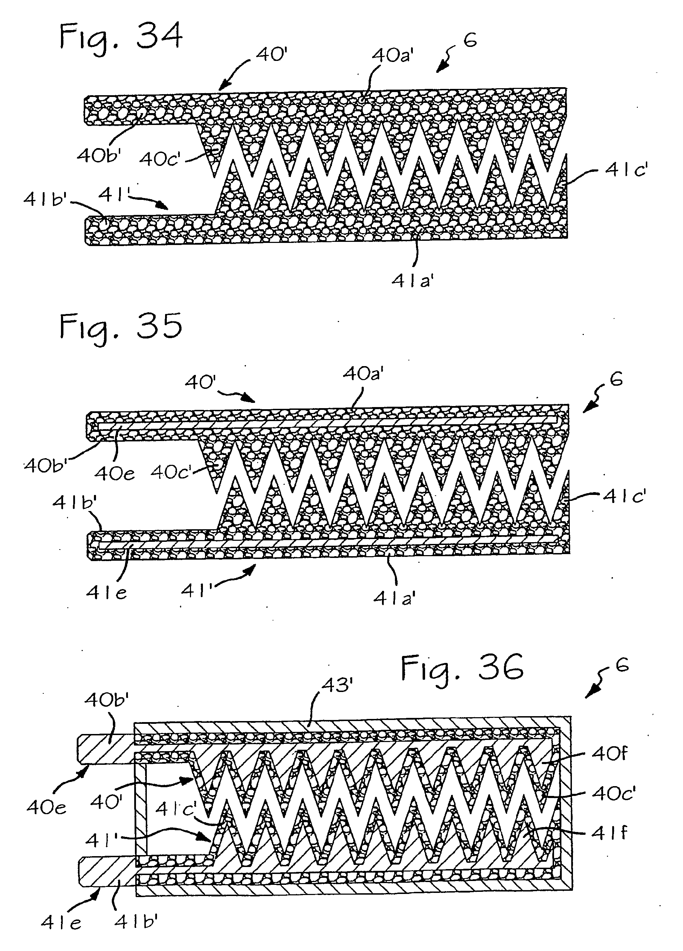

Figure 34 is a schematic illustration of the case of two electrodes for a sensor 6 of the device according to the invention that have a configuration that is substantially similar to that of the electrodes illustrated in Figures 19 and 20. In the case exemplified, said two electrodes, designated by 40' and 41', are obtained via moulding of a synthetic material that is electrically conductive or rendered such, for instance a material with an insulating thermoplastic base to which powder or conductive fibres have been added as fillers.

The electrically conductive thermoplastic materials that can be used to form the electrodes of the device according to the invention have typically a precise value of electrical resistance. Consequently, the greater the length of the electrode, and/or the smaller the respective cross section, the greater the corresponding overall electrical resistance, or rather its different distribution in the different parts of the electrode (in other words, the value of resistance at the tooth 40c', 41c' closest to the terminal 40b', 41b' of the electrode will be decidedly lower than the value of resistance at the tooth furthest from the same terminal). For said reason, it appears preferable to use, for the purposes of formation of the electrodes, conductive synthetic materials with a low resistance and/or with a uniform distribution of said value, in particular for the purpose of preventing anomalous distributions of voltages and/or currents in the electrodes, or else in the respective sensor and/or in the device. The terminals 40b', 41b' are substantially equivalent to the ones described in reference to the preceding examples, or, they could form part of a connector CE.

The special type of moulded electrodes, in particular in the case of electrodes made of electrically conductive thermoplastic material, is suited to obtaining a good adhesion or co-penetration or structural bond with a thermoplastic material that forms the body of the device according to the invention, thus guaranteeing also a good seal with respect to said body. For said purpose, the material of the electrodes and that of at least part of said body are preferably of one and the same type or of a compatible type, for example, one and the same polymer or basic thermoplastic material. In the case of the electrode, said basic material is preferably made with the addition of electrically conductive substances or materials as additives, whilst in the case of the body it is as such or is made with the addition of electrically insulating materials. Merely by way of example, the basic thermoplastic material could be PA66 or PPS, made with the addition of electrically conductive powder or fibre, in the case of use for electrodes, and/or as such or made with the addition of a glass fibre in the case of use for the body of a device and/or of the corresponding connector.

In Figure 35 the case of a sensor 6 is represented, two electrodes 40', 41' of which each have an internal core that is electrically conductive or has a lower electrical resistance, said cores being designated by 40e, 41e and being made, for example, from a chip made of metal material or else a carbon-fibre thread. The core 40e, 41e, obtained with modalities suitable for the purpose, is at least in part coated or overmoulded with an electrically conductive material, preferably of the thermoplastic type, to form the finished electrodes 40', 41', equipped with the respective base parts 40a', 41a', terminal parts 40b', 41b', and teeth 40c', 41c'. Said embodiment enables a better distribution of the current or voltage, via the core with low electrical resistance 40e', 41e', which can be roughly or imprecisely shaped. In use, the current or voltage is then locally distributed by means of the overlying part made of electrically conductive thermoplastic material, having a more precise shape and arrangement, in so far as it is obtained via moulding, enabling, for example, a uniform and/or precise distance or alignment between the surfaces of two facing or opposite electrodes. Said configuration likewise enables reduction of the negative effect due to the resistance of the conductive mouldable material, which, in the case of long paths or electrodes, could create different divisions or variations of the electrical signal. Formation of the electrodes via moulding of electrically conductive synthetic material moreover enables wider plate surfaces (armatures) to be obtained in a more convenient and economic way, even though they are made in the thickness of the respective electrodes, as compared to the case of electrodes formed from metal strap.

Figure 36 illustrates a further version of sensor 6 equipped with electrodes 40', 41' made of electrically conductive synthetic material and each equipped with a core 40e, 41e. In said embodiment, the two conductive cores 40e, 41e of the electrodes themselves have a substantially comblike configuration, presenting for said purpose a plurality of respective projections or teeth 40f, 41f. Conductive thermoplastic material is moulded or applied on the respective core so as to coat the aforesaid projections 40f, 41f, in a position corresponding to which there are thus formed the teeth 40c, 41c of the electrodes 40', 41'. The presence of the projections 40f, 41fofthe cores 40e, 41e enables reduction of the thickness of the electrically conductive overmoulding or coating material, in particular in the area of mutual coupling of the electrodes.

It may be noted that a thin conductive coating for the core could also be obtained, instead of by a moulding operation, by dipping of the core itself in a material (coating) that then hardens. Likewise, the electrically conductive synthetic material could be sprayed on the core 40e, 41e.

As may be appreciated, thanks to the presence of the parts 40f, 41f of the cores 40e, 41e, the respective electrical resistances of the electrodes are even lower than in the case of the embodiment illustrated in Figure 35, and differently or more uniformly distributed, in particular along the entire useful perimeter of the electrode. Said solution contributes to a further improvement in the uniform distribution of the voltages and/or currents and thus to a more precise detection by the sensor.

From Figure 36 it may moreover be noted how the terminals 40b', 41b' of the electrodes 40', 41' are made directly via end portions of the respective cores 40e, 41e, said end portions not being coated with the conductive synthetic material but, for example, being made of metal material and shaped like the terminals of a connector CE. Figure 36 moreover shows a peripheral part 43', made of electrically insulating material, from which the aforesaid terminals 40b', 41b' project at the side. The insulating part 43' can, for example, be a body or frame having the purpose of supporting the electrodes 40', 41, for example as in the case of Figures 19 and 20, or else be a casing of the sensor 6, which defines a chamber in which the detecting part of the electrodes is operatively positioned and which is designed for containment or transit of the fluid being detected, also irrespective of its use in combination with a device of the same type as the ones previously designated by 1, 1', 1". Also in said solution, there could advantageously be envisaged the use of two compatible materials, respectively, for the electrodes and for the body of the device in order to improve mutual fixing and/or seal.

It should be noted that also the solution illustrated in Figure 35 or 36 enables the use of cores 40e, 41e, which do not necessarily have a precise shape and which are made of a metal not necessarily designed to resist a chemical or electrochemical action or aggression, in so far as it is coated with the electrically conductive synthetic material, in particular of the type most resistant to said action. It follows that also the cost of said core is clearly lower than that of an entirely metal electrode, which needs to be precise and/or pre-arranged both for carrying out the functions of detection directly and for withstanding as long as possible said chemical or electrochemical aggression, particularly by the fluid that is being detected.

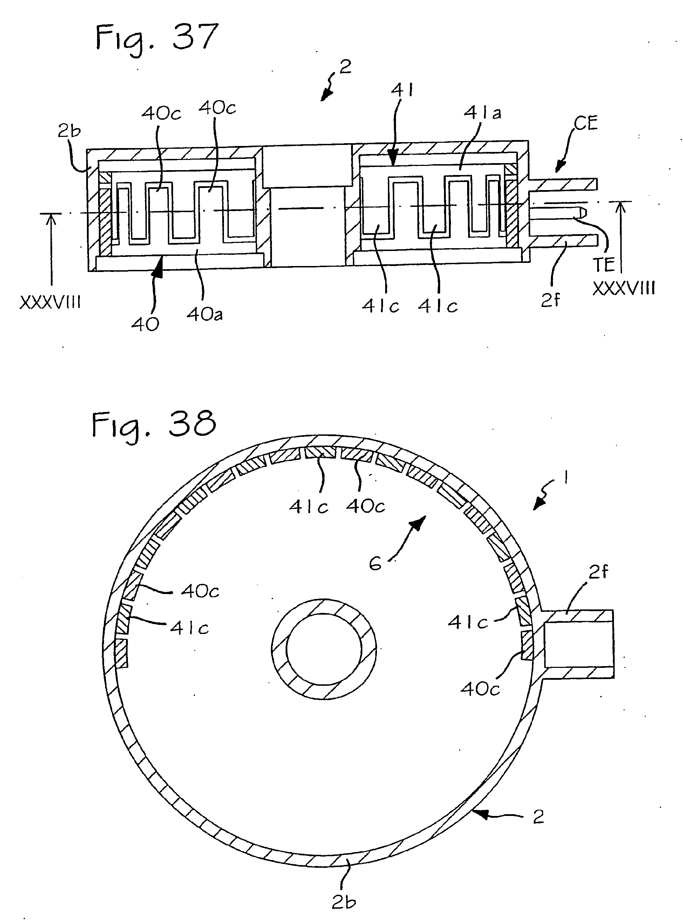

Figures 37 and 38 are, instead, schematic illustrations of the case of a sensor 6 overmoulded on a part of the casing of a device 1 built in a way similar to the one illustrated in Figures 1-2. It may be noted in any case that said solution, like the ones described previously in relation to the embodiment of the electrodes made of electrically conductive synthetic material, may be applied also to devices for detecting conditions of a fluid of a different type. The sensor 6 is in this case overmoulded on the body 2 or co-moulded therewith, and in said embodiment presents a substantially semicircular configuration, with the electrodes 40, 41 overmoulded on the internal surface of the peripheral wall 2b, in relief with respect to the latter. Also in this case the electrodes 40, 41 have a substantially comblike shape, with the respective interdigitated teeth. It may be noted that, in this embodiment, the teeth 40c, 41c of the electrodes are interdigitated in a substantially vertical direction or in a direction parallel to the axis of the device. Instead, in the case of the embodiments illustrated in Figures 1-33, the teeth of the various electrodes are interdigitated in a substantially horizontal direction or in any case in a direction transverse with respect to the longitudinal axis of the device. Obviously said electrodes 40, 41 could also be moulded separately and then associated to the body 2 of the device 1.

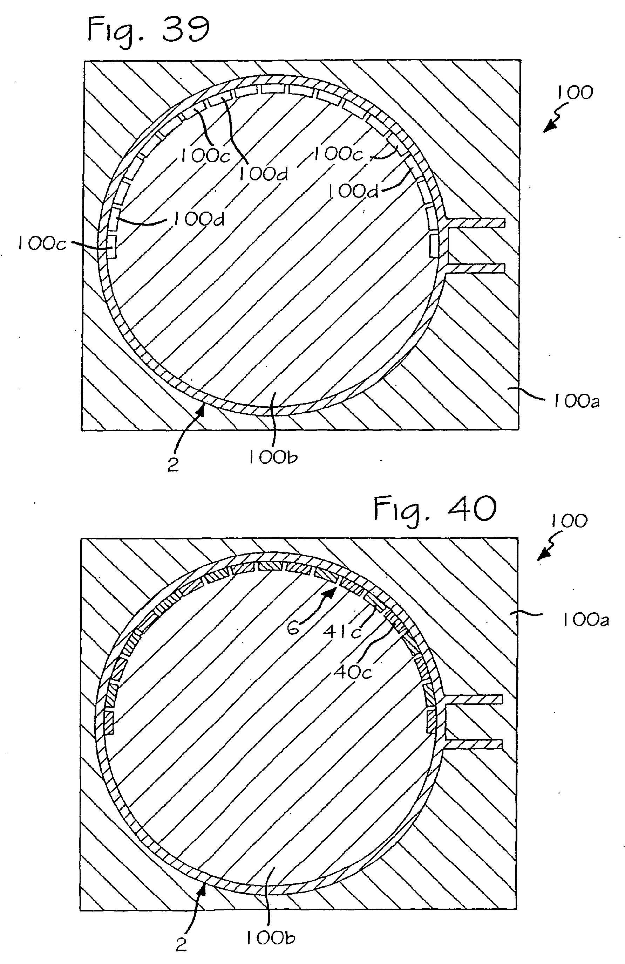

In order to overmould the electrodes 40c, 41c with conductive thermoplastic material a moulding apparatus or mould is used, represented only schematically in Figures 39 and 40. Said mould, designated as a whole by 100, comprises two parts 100a and 100b, of which at least one is movable, said parts being designed to assume, with modalities in themselves known, at least two respective reciprocal positions, i.e., a working position, or closing position, and an opening position. Obviously the mould used could have a geometry different from the one exemplified schematically, according to the conformation of the desired electrodes, or else have a number of movable parts.

The parts 100a, 100b have respective impressions, shaped for defining as a whole a main cavity within the mould 100, when they are closed with respect to one another in the working position, it being possible to house in said main cavity a previously obtained component on which it is desired to overmould the electrodes, such as, for example, the body 2. In this way, two distinct "differential" cavities are defined in the die, i.e., formed by the space of the main cavity not occupied by the body 2. Parts of said cavities, the overall profile of which substantially corresponds to the outer shape of a respective electrode 40, 41 illustrated in Figure 37-38, are designated as a whole by 100c and 100d in Figure 39. Each of said cavities 100c and 100d is provided for receiving electrically conductive thermoplastic material designed to form a respective electrode 40, 41, defining its respective external shape.

A possible moulding sequence in order to obtain the electrodes 40, 41 d Figures 37 and 38 on the body 2, could comprise, for example, the following steps:
i) insertion of the body 2 within the mould part 100a;
ii) closing of the second mould part 100b with respect to the first mould part 100a, under the thrust of respective forces;
iii) introduction of the electrically conductive material, for example, a thermoplastic material with electrically conductive fibre or powder fillers, in an injection duct of the mould, which is in direct communication with the aforesaid differential cavities 100c, 100d, up to filling of the latter;
iv) waiting for enabling cooling and solidification of the electrically conductive thermoplastic material, which thus forms the electrodes 40, 41;
v) opening of the mould parts 100a, 100b, which are moved away from one another via respective tensile forces;
vii) extraction of the body 2 from one of the mould parts 100a, 100b.

Preferably, in order to overmould or co-mould the electrodes 40, 41, in the basic component (the body 2, in the example), appropriate seats, channels or passages are formed, which are to receive anchoring parts of conductive thermoplastic material that forms the overmoulded components, i.e., the electrodes and/or the connector CE, said parts being operative for withholding the components themselves in position. Said channels could moreover enable or facilitate a further step, for example, comprised between the aforesaid steps (i) and (ii), during which in the mould and/or in said channels also a possible core made of electrically conductive material is inserted.

From the foregoing description there clearly emerge the characteristics of the present invention, as likewise there clearly emerge its advantages.

In accordance with a first aspect of the invention, the fact that the detection device is equipped with joint means that are the same as the ones of the filter with which it is designed to co-operate means that it can be readily inserted into and/or removed from the hydraulic system of a vehicle, without this entailing particular difficulties of assembly/disassembly and/or of operation of the system; or, it enables installation of just the filter without the detecting device, or else mounting of the detecting device at a later date, connecting up on the hydraulic joint provided for a filter in any case present on the vehicle. As has been said, the device according to the invention can possibly function not only as a detector of characteristics of the fluid, but also as an adapter in order to enable installation on a vehicle of a filter that is commercially available but not originally envisaged for that type of vehicle, or, it is provided with a different attachment.

The device is inexpensive to produce as compared to currently available detection sensors, in so far as it can be readily subjected to periodic maintenance or replacement, in particular during the normal operations of periodic replacement of the filter. For this purpose, the detection device can be easily replaced if need be, or else can be momentarily dismantled to undergo washing aimed at restoring its proper operation. Said possibility of ease of maintenance also enables production of the detection device with technologies and/or materials of lower cost as compared to known sensors, which must, instead, necessarily guarantee a longer duration, i.e., the entire life of the vehicle without any maintenance.

In accordance with a second aspect of the invention, the possibility of obtaining a generic detection device equipped with sensor means for detecting electrical or physical quantities of the fluid which have at least one electrode made of electrically conductive synthetic material enables simplification from the standpoint of production of the device itself, and hence reduction of the corresponding costs. Said characteristic likewise enables an increase in the duration of useful life of the detection device, in so far as said electrodes are less subject to any aggression of a chemical and/or electrochemical nature by the fluid undergoing detection.

Of course, without prejudice to the principles of the invention, the components and materials used and the shapes and/or processes adopted may vary from the ones described and illustrated herein. Furthermore, the individual components described may be produced or obtained with any other known technique and may in part be omitted, or else be present in a different number and with a different arrangement, or else at least in part be combined together in order to pursue the purposes of the present invention.

As has been said, in addition to the detection of intrinsic characteristics of the fluid forming the subject of analysis, the device according to the invention can advantageously integrate means designed to monitor the operating characteristics of the filter, such as a pressure sensor.

The electronic-control circuit integrated in the device 1',1" could be of the type designed to transmit and/or receive data via a wireless communication system to or from a second control circuit located in a remote position, for example, in the passenger compartment of the vehicle, for the purpose of signalling and/or displaying to the driver the state of the fluid and/or of the filter, or else be associated to other electronic control units of the vehicle. Said variant presents the advantage of enabling an ease of installation of the device according to the invention, even on vehicles that were not previously equipped therewith, or else on which only a filter mounted directly on the engine is present.

With reference to the sensor means of the type designated by 6, 6', 6", the electrodes could face at least in part the respective plane surfaces of larger size, instead of the respective surfaces of smaller size, defined by the thickness of the original lamina or strap, or else they could be of shapes and/or materials that are completely different, albeit suitable for the purpose. In other variant embodiments, the sensor means could comprise electrodes with parts that are substantially the same as one another and/or parallel to one another, for example, at least in part superimposed, also having a plane, straight or square configuration, or any other shape suited to the purpose.

The electrodes of the sensor means could also be made with other moulding or forming technologies, for example, via sintering or other technologies in any case designed to form the electrodes themselves, in particular starting from fibres and/or powder of electrically conductive materials.

In possible alternative configurations of the device according to the invention, the functional component previously identified as sleeve 4', 4" could be integrated, i.e., made of a single piece, with one of the components 2', 2" or 3', 3" of the body of the device.

The device forming the subject of the invention may be made by associating and/or combining together at least part of the elements and/or teachings described in reference to the various figures or variants, previously provided by way of non-limiting example.

The invention has been described, by way of non-limiting example, with reference to its use in combination with a hydraulic circuit or a filter for a lubricating fluid. It is, however, clear that the invention may be used to advantage in circuits of a different type, and in particular circuits of a vehicle in which a fluid or liquid of a different type is present, such as a fuel, a fluid additive, a conditioning liquid, a washing liquid, a fluid of a braking system, etc.

## Claims

1. A device for detecting at least a condition of a fluid, designed for use on a vehicle having a hydraulic circuit that comprises at least one filtering member (20) having an outlet joint (23a), designed for coupling with an inlet joint (32) provided in a fixed part (30) of the vehicle, the device (1'; 1") comprising detecting means for detecting at least a condition of the fluid (6'-8', 70; 6"-8", 70"), said device (1'; 1") comprising a respective body (2', 3', 4'; 2", 3", 4") that is independent with respect to said filtering member (20) and to said fixed part (30) of the vehicle and includes a connection element (4'; 4") that has first connection means (4b', 4g'; 4b", 4g"), adapted to couple to said outlet joint (23a) of the filtering member (20), and second connection means (4a', 4f; 4a", 4f'), adapted to couple to said inlet joint (32) of the fixed part (30) of the vehicle, said body (2', 3', 4'; 2", 3", 4") delimiting at least one respective detection chamber (C'; C"), to which said detecting means (6'-8', 70; 6"-8", 70") are operatively associated, such that the device (1'; 1") can be operatively arranged in a removable way between said filtering member (20) and said fixed part (30) of the vehicle,
wherein said connection element (4'; 4") has an internal conduit (4d', 4e'; 4d", 4e") for the fluid, said conduit (4d', 4e'; 4d", 4e") having an upper portion (4e'; 4e"), for connection to said outlet joint (23a) of the filtering member (20), and a lower portion (4d'; 4d"), for connection to said inlet joint (32) of the fixed part (30) of the vehicle,
and wherein said detecting means comprise electric sensor means (6'-8', 70; 6"-8", 70") with which the fluid comes into contact,
**characterized in that**
- said upper portion (4e'; 4e") and said lower portion (4d'; 4d") of said conduit are separated from one another by means of an intermediate restriction (4p', 4pp'; 4p", 4pp") or by an intermediate closed wall;
- said connection element (4'; 4") has at least one first through hole (4r; 4r") and at least one second through hole (4q; 4q") in communication with said detection chamber (C'; C"), said first through hole (4r; 4r") and second through hole (4q; 4q") being upstream and downstream of said intermediate restriction or intermediate closed wall (4p', 4pp'; 4p", 4pp"), respectively, such that first through hole (4r; 4r") enables passage of the fluid from said upper portion (4e'; 4e") of said conduit into said detection chamber (C'; C") and the second through hole (4q; 4q") enables passage of the fluid from said detection chamber (C'; C") to said lower portion (4d'; 4d") of said conduit,
in such a way that detections performed through said sensor means (6'-8', 70; 6"-8", 70") are carried out on the fluid already filtered by said filtering member (20).

2. Device according to Claim 1, **characterized in that** said first connection means (4b', 4g'; 4b", 4g") and said second connection means (4a', 4f; 4a", 4f") comprise respective first and second mechanical connection means (4f', 4g'; 4f', 4g") and/or respective first and second hydraulic connection means (4a', 4b'; 4a", 4b").

3. Device according to Claim 2, **characterized in that** the first mechanical connection means and the second mechanical connection means comprise at least one of a male attachment (4f'; 4f') and a female attachment (4g'; 4g").

4. Device according to Claim 3, **characterized in that** the male attachment comprises an externally threaded tubular part and the female attachment comprises an internally threaded tubular part.

5. Device according to at least one of the preceding claims, **characterized in that** said body (2', 3', 4'; 2", 3", 4") comprises a positioning seat (2c'; 3c'; 81) for said connection element (4'; 4").

6. Device according to claim 1, **characterized in that** said connection element (4'; 4") is made of a single piece with one component (2', 2"; 3', 3") of said body (2', 3', 4'; 2", 3", 4").

7. The device according to Claim 1, **characterized in that** said electric sensor means (6'-8', 70; 6"-8", 70") comprise at least one from among:
- sensor means (6', 6") for detecting an electrical or physical quantity, such as a value of conductivity, resistivity, capacitance, impedance, acidity;
- temperature-sensing means (7'; 7"), such as a negative-temperature-coefficient resistor;
- fluidity-sensing means (8'; 8"), such as a quartz oscillator;
- pressure-sensing means (70; 70").

8. Device according to Claim 7, **characterized in that** said sensor means for detecting an electrical or physical quantity comprise a sensor (6', 6") having at least one electrode (40, 41; 40, 41, 42; 42, 44; 40', 41') that is to come into contact with the fluid present in said detection chamber (C'; C").

9. Device according to Claim 8, **characterized in that** a plurality of said sensors (6a, 6b, 6c) is operative in said detection chamber (C'; C"), where in particular at least two of said sensors (6a, 6b, 6c) have an electrode in common.

10. Device according to Claim 8, **characterized in that** a support means (43; 43') is provided for one or more of said electrodes (40, 41; 40, 41, 42; 42, 44; 40', 41'), in order to keep them in a fixed position.

11. Device according to one or more of the preceding claims, **characterized in that** one or more of said detecting means (70; 70") are housed at least in part in a respective housing space (70a) in hydraulic communication with said detection chamber (C'; C").

12. Device according to Claim 7, **characterized in that** said pressure-sensing means comprise a pressure sensor (70; 70") of a differential type.

13. Device according to Claim 11 or 12, **characterized in that** said pressure-sensing means (70; 70") are connected to said detection chamber (C'; C") and/or to a further chamber (73) distinct from said detection chamber (C'; C").

14. Device according to Claim 1, **characterized in that** in said body (2', 3', 4'; 2", 3", 4") a subchamber or housing chamber (70a) is delimited, hermetically isolated from said detection chamber (C'; C").

15. Device according to Claim 1, **characterized in that** it has a structure that is at least in part modular or adaptable, in order to allow to obtain, using a limited number of basic components, a multiplicity of different versions of the device itself.

16. Device according to Claim 5, **characterized in that** said body (2', 3', 4'; 2", 3", 4") comprises at least two components (2', 3') coupled to one another, at least one component comprising at least one portion or part of said positioning seat (2c'; 3c').

17. Device according to Claim 5, **characterized in that** said body (2', 3', 4'; 2", 3", 4") comprises at least two components (2", 3") coupled to one another, one of said components (3") forming entirely said positioning seat (81).

18. Device according to at least one of Claims 4 and 5, **characterized in that** said body comprises at least two components coupled to one another, at least one of said components (2', 3', 4'; 2", 3", 4") integrating at least in part said connection element.

19. Device according to at least one of Claims 16 to 18, **characterized in that**
- said components (2', 3'; 2", 3") are joined together via welding or thermofusion or bonding, or else via mechanical-coupling means, such as a coupling of a female-thread/male-thread type, a press-block coupling, a bayonet coupling, a snap-action coupling, and/or
- said components (2', 3'; 2", 3") are joined together in at least one region of mutual coupling in a peripheral area or wall (2b, 3b) and/or an internal area or wall (3c, 3c).

20. Device according to Claim 1, **characterized in that** it comprises an electrical connector (CE), formed at least in part by said body (2', 3'; 2", 3") or by a component associated thereto.

21. Device according to Claim 1, **characterized in that** it comprises an electronic circuit (60).

22. Device according to Claim 21, **characterized in that** said electronic circuit (60) comprises at least one from among:
- a measurement circuit, in particular for generating and/or detecting electrical signals;
- a digital processor or microprocessor or microcontroller;
- electronic memory means;
- an amplification circuit, preferably of a type programmable at least for the respective gain;
- a circuit for calibration, of automatic type or of the type designed to vary a digital datum or the value of a component, such as, for example, a resistor;
- an electronic switch;
- a device for control or actuation or selection or switching;
- an electric-power supply circuit, and
- communication means, in particular comprising at least one of a line for wired communication of signals and a circuit for wireless communication of signals.

23. Device according to Claim 8, **characterized in that** said electrode or electrodes (40, 41; 40, 41, 42; 42, 44; 40', 41') are made of metal, in particular obtained starting from metal strap, preferably of small thickness.

24. Device according to Claim 8, **characterized in that** said electrode or electrodes (40, 41; 40, 41, 42; 42, 44; 40', 41') are formed at least in part with a synthetic material that is electrically conductive or rendered such, such as a thermoplastic material with the addition of electrically conductive material as a filler, where in particular said electrode or electrodes (40, 41; 40, 41, 42; 42, 44; 40', 41') are formed at least in part via moulding or silk-screen printing.

25. Device according to Claim 24, **characterized in that** said electrode or electrodes (40', 41') comprise an electrically conductive core (40e, 41e) coated at least partially with said electrically conductive synthetic material, in particular overmoulded or deposited.

26. Device according to Claim 24, **characterized in that** said electrically conductive synthetic material is overmoulded on, or distributed, or silk-screen printed on at least part of a component of the device itself, or vice versa, or co-moulded with at least part of said component, where in particular one or more seats, channels or passages are formed in said component (2), to receive said electrically conductive synthetic material and/or anchoring parts for the latter.

## Patentansprüche

1. Vorrichtung zum Erfassen von wenigstens einem Zustand eines Fluids, vorgesehen zur Verwendung an einem Fahrzeug mit Hydraulikkreislauf, wobei wenigstens ein Filterglied (20) mit einem Auslassanschluss (23a), bestimmt zum Koppeln mit einem an einem fixierten Teil (30) des Fahrzeugs vorgesehenen Einlassanschluss (32) vorgesehen ist, wobei die Vorrichtung (1'; 1") Sensoren (6'-8', 70; 6"-8", 70") zum Erfassen wenigstens eines Fluidzustandes aufweist, wobei die Vorrichtung (1'; 1") einen Körper (2', 3', 4'; 2", 3", 4") aufweist, welcher unabhängig ist in Bezug auf das Filterglied (20) und auf das fixierte Teil (30) des Fahrzeuges und ein Verbindungselement (4'; 4") enthält, welches erste Verbhdungseinrichtungen (4b', 4g'; 4b", 4g") aufweist, welche geeignet sind mit dem Auslassanschluss (23a) des Filtergliedes (20) gekoppelt zu werden und zweite Anschlussglieder (4a', 4f'; 4a", 4f"), geeignet zum Koppeln mit dem Einlassanschluss (32) des fixierten Teils (30) des Fahrzeuges, wobei der Körper (2', 3', 4'; 2", 3", 4") wenigstens eine Erfassungskammer (C'; C") begrenzt, welcher die Sensoren (6'-8', 70; 6"-8", 70") operativ zugeordnet sind, derart, dass die Vorrichtung (1', 1") operativ auf entnehmbare Weise zwischen dem Filterglied (20) und dem fixierten Teil (30) des Fahrzeuges vorgesehen werden kann, wobei das Verbindungselement (4', 4") eine interne Leitung (4d', 4e'; 4d", 4e") für das Fluid aufweist, wobei diese Leitung (4d', 4e'; 4d", 4e") einen oberen Abschnitt (4e'; 4e") zum Verbinden mit dem Auslassanschluss (23a) des Filtergliedes (20) und einen unteren Abschnitt (4d'; 4d") zum Verbinden mit der inneren Leitung (32) des fixierten Teils (30) des Fahrzeuges aufweist,
und wobei die Sensoren elektrische Sensoren (6'-8', 70; 6"-8", 70") sind, mit welchen das Fluid in Berührung gelangt,
**dadurch gekennzeichnet, dass**
- der obere Abschnitt (4e'; 4e") und der untere Abschnitt (4d'; 4d") der Leitung voneinander getrennt sind mittels einer Zwischeneinschnürung (4p', 4pp'; 4p", 4pp") oder mittels einer geschlossenen Zwischenwand;
- wobei das Verbindungselement (4'; 4") wenigstens eine erste Durchgangsbohrung (4r, 4r") und wenigstens eine zweite Durchgangsbohrung (4q; 4q") in Verbindung mit der Erfassungskammer (C'; C") aufweist, wobei die erste Durchgangsbohrung (4r; 4r") und die zweite Durchgangsbohrung (4q; 4q") strömungsaufwärts und strömungsabwärts von der Zwischeneinschnürung oder der geschlossenen Zwischenwand (4p', 4pp'; 4p", 4pp") jeweils angeordnet sind, derart, dass die erste Durchgangsbohrung (4r; 4r") den Durchtritt von Fluid aus dem oberen Abschnitt (4e'; 4e") der Leitung in die Erfassungskammer (C'; C") ermöglicht und die zweite Durchgangsbohrung (4q; 4q") den Durchtritt von Fluid aus der Erfassungskammer (C'; C") zu dem unteren Abschnitt (4d'; 4d") der Leitung ermöglicht,
und zwar derart, dass die mit Hilfe der Sensoren (6'-8', 70; 6"-8", 70") durchgeführten Ermittlungen an Fluid ausgeführt werden, welches bereits von dem Filterglied 20 gefiltert ist.

2. Vorrichtung nach Anspruch 1, **dadurch gekennzeichnet, dass** die erste Verbindungseinrichtung (4b', 4g'; 4b", 4g") und die zweite Verbindungseinrichtung (4a', 4f'; 4a", 4f") jeweils erste und zweite mechanische Verbinder (4f', 4g'; 4f", 4g") und/oder jeweils erste und zweite Hydraulikverbinder (4a', 4b'; 4a", 4b") aufweisen.

3. Vorrichtung nach Anspruch 2, **dadurch gekennzeichnet, dass** der erste mechanische Verbinder und der zweite mechanische Verbinder jeweils wenigstens eh Steckerteil (4f'; 4f") oder ein Buchsenteil (4g', 4g") aufweisen.

4. Vorrichtung nach Anspruch 3, **dadurch gekennzeichnet, dass** das Steckerteil ein rohrförmiges Teil mit Außengewinde und das Buchsenteil ein rohrförmiges Teil mit Innengewinde aufweist.

5. Vorrichtung nach wenigstens einem der vorangehenden Ansprüche, **dadurch gekennzeichnet, dass** der Körper (2', 3', 4'; 2", 3", 4") einen Positionierungssitz (2c', 3c'; 81) für das Verbindungselement (4', 4") aufweist.

6. Vorrichtung nach Anspruch 1, **dadurch gekennzeichnet, dass** das Verbindungselement (4', 4") einstückig ist mit einer Komponente (2', 2";:3', 3") des Körpers (2', 3', 4'; 2", 3", 4").

7. Vorrichtung nach Anspruch 1, **dadurch gekennzeichnet, dass** die elektrischen Sensoren (6'-8', 70; 6"-8", 70") wenigstens eines der folgenden Bauteile aufweisen:
- eine Sensoreinrichtung (6', 6") zum Erfassen einer elektrischen oder physikalischen Quantität, wie der Leitfähigkeit, des Widerstandes, der Kapazität, der Impedanz oder Azidität;
- Temperaturfühler (7'; 7"), wie einen Negativ-Temperaturkoeffizienzresistor;
- Fluiditätsfühler (8'; 8"), wie einen Quarzoszillator;
- Druckfühler (70; 70").

8. Vorrichtung nach Anspruch 7, **dadurch gekennzeichnet, dass** die Sensoreinrichtung zum Erfassen einer elektrischen oder physikalischen Quantität einen Sensor (6', 6") mit wenigstens einer Elektrode (40, 41; 40, 41, 42; 42, 44; 40', 41') aufweist, die in Kontakt mit dem in der Erfassungskammer (C'; C") vorliegenden Fluid bringbar ist.

9. Vorrichtung nach Anspruch 8, **dadurch gekennzeichnet, dass** eine Vielzahl jener Sensoren (6a, 6b, 6c) in der Erfassungskammer (C'; C") operativ ist, wobei insbesondere zwei dieser Sensoren (6a, 6b, 6c) eine gemeinsame Elektrode aufweisen.

10. Vorrichtung nach Anspruch 8, **dadurch gekennzeichnet, dass** eine Stützeinrichtung (43; 43') für eine oder mehrere der Elektroden (40, 41; 40, 41, 42; 42, 44; 40', 41') vorgesehen ist, um dieselben in einer fixierten Position zu halten.

11. Vorrichtung nach einem oder mehreren der vorangehenden Ansprüche, **dadurch gekennzeichnet, dass** wenigstens eine der Erfassungseinrichtungen (70; 70") wenigstens zum Teil in einem entsprechenden Gehäusespalt (70a) in hydraulischer Verbindung mit der Erfassungskammer (C'; C") untergebracht ist.

12. Vorrichtung nach Anspruch 7, **dadurch gekennzeichnet, dass** die Druckfühler einen Drucksensor (70; 70") vom Differentialtyp aufweisen.

13. Vorrichtung nach Anspruch 11 oder 12, **dadurch gekennzeichnet, dass** der Drucksensor (70; 70") an die Erfassungskammer (C', C") und/oder an eine weitere Kammer (73) angeschlossen ist, welche von der Erfassungskammer (C'; C") verschieden ist.

14. Vorrichtung nach Anspruch 1, **dadurch gekennzeichnet, dass** in dem Körper (2', 3', 4'; 2", 3", 4") eine Unterkammer oder Gehäusekammer (70a) hermetisch isoliert gegen die Erfassungskammer (C'; C") abgegrenzt ist.

15. Vorrichtung nach Anspruch 1, **dadurch gekennzeichnet, dass** sie eine Struktur aufweist, welche wenigstens zum Teil modular oder adaptierbar ist, um es zu ermöglichen, mit einer begrenzte Anzahl von Basiskomponenten, eine Vielzahl von unterschiedlichen Versionen der Vorrichtung zu erhalten.

16. Vorrichtung nach Anspruch 5, **dadurch gekennzeichnet, dass** der Körper (2', 3', 4'; 2", 3", 4") wenigstens zwei Komponenten (2', 3') aufweist, welche miteinander gekoppelt sind, wobei wenigstens eine Komponente wenigstens einen Abschnitt oder einen Teil des Positionierungssitzes (2c', 3c') aufweist.

17. Vorrichtung nach Anspruch 5, **dadurch gekennzeichnet, dass** der Körper (2', 3', 4'; 2", 3", 4") wenigstens zwei Komponenten (2", 3") aufweist, welche miteinander gekoppelt sind, wobei eine der Komponenten (3") vollständig den Positionierungssitz (81) bildet.

18. Vorrichtung nach Anspruch 4 oder 5, **dadurch gekennzeichnet, dass** der Körper wenigstens zwei miteinander gekoppelte Komponenten aufweist, wobei wenigstens eine dieser Komponenten (2', 3', 4'; 2", 3", 4") wenigstens zum Teil in das Verbindungselement integriert ist.

19. Vorrichtung nach wenigstens einem der Ansprüche 16 bis 18, **dadurch gekennzeichnet, dass**
- die Komponenten (2', 3'; 2", 3") mittels Schweißen oder Thermofusion oder Bonding miteinander verbunden sind, oder mittels mechanischer Kopplungsmöglichkeiten, wie einer Verbindung vom Stecker-Buchse-Typ, einer Pressblock-Verbindung, eines Bajonettverschlusses, einer Einschnappverbindung, und/oder
- die Komponenten (2', 3'; 2", 3") miteinander in wenigstens einem Bereich einer gegenseitigen Verbindung in einem Umfangsbereich oder einer Wandung (2b, 3b) und/oder in einem Innenbereich oder Innenwand (3c, 3c) miteinander verbunden sind.

20. Vorrichtung nach Anspruch 1, **dadurch gekennzeichnet, dass** sie einen elektrischen Verbinder (CE) aufweist, welcher wenigstens zum Teil durch den Körper (2', 3; 2", 3") oder durch eine damit verbundene Komponente gebildet ist.

21. Vorrichtung nach Anspruch 1, **dadurch gekennzeichnet, dass** sie einen elektronischen Schaltkreis (60) umfasst.

22. Vorrichtung nach Anspruch 21, **dadurch gekennzeichnet, dass** der elektronische Schaltkreis (60) wenigstens eine der folgenden Schaltkreise umfasst:
- einen Messkreis, insbesondere zum Erzeugen und/oder Erfassen von elektrischen Signalen,
- einen Digitalprozessor oder Mikroprozessor oder Mikrocontroller,
- elektronische Memory-Einrichtung,
- einen Verstärkerkreis, vorzugsweise von einem wenigstens für das jeweilige Ziel programmierbare Typ,
- einen Kalibrationsschaltkreis vom automatischen Typ oder von einem zur Variation eines digitalen Datums oder des Wertes einer Komponente, wie z. B. eines Resistors bestimmten Typs,
- einen elektronischen Schalter, eine Vorrichtung zum Steuern oder Betätigen oder Auswählen oder Schalten,
- einen elektrischen Leistungsversorgungsschaltkreis, und
- Kommunikationseinrichtungen, insbesondere enthaltend wenigstens eine Leitung für leitungsgebundene Signalkommunikation oder einen Schaltkreis für die drahtlose Signalkommunikation.

23. Vorrichtung nach Anspruch 8, **dadurch gekennzeichnet, dass** die Elektrode oder Elektroden (40, 41; 40, 41, 42;42, 44; 40', 41') aus Metall bestehen, insbesondere ausgehend von Metallband vorzugsweise geringer Dicke.

24. Vorrichtung nach Anspruch 8, **dadurch gekennzeichnet, dass** die Elektrode oder die Elektroden (40, 41; 40, 41, 42; 42, 44; 40', 41') wenigstens zum Teil aus einem synthetischen Material hergestellt sind, welches elektrisch leitend ist oder leitend gemacht ist, wie aus einem thermoplastischen Material unter Zusatz von elektrisch leitendem Füllmaterial, wobei die Elektrode oder die Elektroden (40, 41; 40, 41, 42; 42, 44; 40', 41') vorzugsweise wenigstens zum Teil durch Moulding oder Siebdruck hergestellt sind.

25. Vorrichtung nach Anspruch 24, **dadurch gekennzeichnet, dass** die Elektrode oder die Elektroden (40', 41') einen elektrisch leitenden Kern (40e, 41 e) aufweisen, welcher wenigstens teilweise mit dem elektrisch leitenden synthetischen Material beschichtet ist, insbesondere durch Overmoulden oder Abscheiden.

26. Vorrichtung nach Anspruch 24, **dadurch gekennzeichnet, dass** das elektrisch leitende synthetische Material overmouldet ist auf, verbreitet ist über oder mittels Siebdruck aufgedruckt ist auf wenigstens einen Teil einer Komponente der Vorrichtung selbst oder vice versa oder comouldet ist mit wenigstens einem Teil der Komponente, wobei insbesondere ein Sitz oder mehrere Sitze, Kanäle oder Passagen in der Komponente (2) ausgebildet sind, um das elektrisch leitende synthetische Material und/oder Ankerteile für letztere aufzunehmen.

## Revendications

1. Dispositif pour détecter au moins un état d'un fluide, conçu pour être utilisé sur un véhicule ayant un circuit hydraulique qui comprend au moins un élément de filtration (20) ayant un joint de sortie (23a) conçu pour se coupler avec un joint d'entrée (32) prévu dans une partie fixe (30) du véhicule, le dispositif (1' ; 1") comprenant des moyens de détection pour détecter au moins une condition du fluide (6' -8' , 70 ; 6"-8", 70"), le dispositif (1'; 1") comprenant un corps (2', 3', 4' ; 2", 3", 4") respectif qui est indépendant par rapport audit élément de filtration (20) et par rapport à ladite partie fixe (30) du véhicule et comprend un élément de raccordement (4' ; 4") qui a des premiers moyens de raccordement (4b', 4g' ; 4b", 4g") adaptés pour se coupler audit joint de sortie (23a) de l'élément de filtration (20) et des seconds moyens de raccordement (4a', 4f'; 4a", 4f") adaptés pour se coupler audit joint d'entrée (32) de la partie fixe (30) du véhicule, ledit corps (2', 3', 4' ; 2", 3", 4") délimitant au moins une chambre de détection (C' ; C") respective à laquelle lesdits moyens de détection (6'-8', 70 ; 6"-8", 70") sont associés de manière opérationnelle, de sorte que le dispositif (1' ; 1") peut être agencé de manière opérationnelle d'une manière amovible entre ledit élément de filtration (20) et ladite partie fixe (30) du véhicule,
dans lequel ledit élément de raccordement (4' ; 4") a un conduit interne (4d', 4e' ; 4d", 4e") pour le fluide, ledit conduit (4d', 4e' ; 4d", 4e") ayant une partie supérieure (4e' ; 4e") pour le raccordement audit joint de sortie (23a) de l'élément de filtration (20), et une partie inférieure (4d' ; 4d") pour le raccordement dudit joint d'entrée (32) de la partie fixe (30) du véhicule,
et dans lequel lesdits moyens de détection comprennent des moyens de capteur électrique (6'-8', 70 ; 6"-8", 70") avec lesquels le fluide vient en contact,
**caractérisé en ce que**
- ladite partie supérieure (4e' ; 4e") et ladite partie inférieure (4d' ; 4d") dudit conduit sont séparées l'une de l'autre au moyen d'une restriction intermédiaire (4p', 4pp' ; 4p", 4pp") ou par une paroi fermée intermédiaire ;
- ledit élément de raccordement (4' ; 4") a au moins un premier trou débouchant (4r ; 4r") et au moins un second trou débouchant (4q ; 4q") en communication avec ladite chambre de détection (C'; C"), ledit premier trou débouchant (4r ; 4r") et ledit second trou débouchant (4q ; 4q") étant en amont et en aval de ladite restriction intermédiaire ou paroi fermée intermédiaire (4p', 4pp' ; 4p", 4pp") respectivement, de sorte qu'un premier trou débouchant (4r ; 4r") permet le passage du fluide à partir de ladite partie supérieure (4e' ; 4e") dudit conduit dans ladite chambre de détection (C' ; C") et le second trou débouchant (4q ; 4q") permet le passage du fluide à partir de ladite chambre de détection (C' ; C") jusqu"à ladite partie inférieure (4d' ; 4d") dudit conduit,
de sorte que les détections réalisées par lesdits moyens de capteur (6'-8', 70 ; 6"-8", 70") sont réalisées sur le fluide déjà filtré par ledit élément de filtration (20).

2. Dispositif selon la revendication 1, **caractérisé en ce que** lesdits premiers moyens de raccordement (4b', 4g' ; 4b", 4g") et lesdits seconds moyens de raccordement (4a', 4f' ; 4a", 4f") comprennent des premier et second moyens de raccordement mécaniques (4f', 4g' ; 4f", 4g") respectifs et/ou des premier et second moyens de raccordement hydrauliques (4a', 4b' ; 4a", 4b") respectifs.

3. Dispositif selon la revendication 2, **caractérisé en ce que** les premiers moyens de raccordement mécaniques et les seconds moyens de raccordement mécaniques comprennent au moins l'un parmi une fixation mâle (4f' ; 4f") et une fixation femelle (4g' ; 4g").

4. Dispositif selon la revendication 3, **caractérisé en ce que** la fixation mâle comprend une partie tubulaire extérieurement filetée et la fixation femelle comprend une partie tubulaire intérieurement filetée.

5. Dispositif selon au moins l'une des revendications précédentes, **caractérisé en ce que** ledit corps (2', 3', 4' ; 2", 3", 4") comprend un siège de positionnement (2c' ; 3c' ; 81) pour ledit élément de raccordement (4' ; 4").

6. Dispositif selon la revendication 1, **caractérisé en ce que** ledit élément de raccordement (4' ; 4") est réalisé d'un seul tenant avec un composant (2', 2" ; 3', 3") dudit corps (2', 3', 4' ; 2", 3", 4").

7. Dispositif selon la revendication 1, **caractérisé en ce que** lesdits moyens de capteur électriques (6'-8', 70 ; 6"-8", 70") comprennent au moins l'un parmi :
- des moyens de capteur (6', 6") pour détecter une quantité électrique ou physique, telle qu'une valeur de conductivité, de résistivité, de capacitance, d'impédance, d'acidité ;
- des moyens de détection de température (7' ; 7") tels qu'une résistance à coefficient de température négatif ;
- des moyens de détection de fluidité (8' ; 8") tels qu'un oscillateur à cristal ;
- des moyens de détection de pression (70 ; 70").

8. Dispositif selon la revendication 7, **caractérisé en ce que** lesdits moyens de capteur pour détecter une quantité électrique ou physique comprennent un capteur (6', 6") ayant au moins une électrode (40, 41 ; 40, 41, 42 ; 42, 44 ; 40', 41') qui doit venir en contact avec le fluide présent dans ladite chambre de détection (C' ; C").

9. Dispositif selon la revendication 8, **caractérisé en ce qu'**une pluralité desdits capteurs (6a, 6b, 6c) est opérationnelle dans ladite chambre de détection (C' ; C"), où en particulier au moins deux desdits capteurs (6a, 6b, 6c) ont une électrode en commun.

10. Dispositif selon la revendication 8, **caractérisé en ce qu'**un moyen de support (43 ; 43') est prévu pour une ou plusieurs desdites électrodes (40, 41 ; 40, 41, 42 ; 42, 44 ; 40', 41') afin de les maintenir dans une position fixe.

11. Dispositif selon une ou plusieurs des revendications précédentes, **caractérisé en ce qu'**un ou plusieurs desdits moyens de détection (70 ; 70") sont logés au moins en partie dans un espace de logement (70a) respectif en communication hydraulique avec ladite chambre de détection (C' ; C").

12. Dispositif selon la revendication 7, **caractérisé en ce que** lesdits moyens de détection de pression comprennent un capteur de pression (70 ; 70") d'un type différentiel.

13. Dispositif selon la revendication 11 ou 12, **caractérisé en ce que** lesdits moyens de détection de pression (70 ; 70") sont raccordés à ladite chambre de détection (C' ; C") et/ou à une autre chambre (73) différente de ladite chambre de détection (C' ; C ").

14. Dispositif selon la revendication 1, **caractérisé en ce que** dans ledit corps (2', 3', 4' ; 2", 3", 4"), est délimitée une sous-chambre ou chambre de logement (70a) hermétiquement isolée de ladite chambre de détection (C' ; C").

15. Dispositif selon la revendication 1, **caractérisé en ce qu'**il a une structure qui est au moins en partie modulaire ou adaptable afin de permettre l'obtention, à l'aide d'un nombre limité de composants de base, d'une multiplicité de versions différentes du dispositif lui-même.

16. Dispositif selon la revendication 5, **caractérisé en ce que** ledit corps (2', 3', 4' ; 2", 3", 4") comprend au moins deux composants (2', 3') couplés entre eux, au moins un composant comprenant au moins une portion ou partie dudit siège de positionnement (2c' ; 3c').

17. Dispositif selon la revendication 5, **caractérisé en ce que** ledit corps (2' , 3', 4' ; 2", 3", 4") comprend au moins deux composants (2", 3") couplés entre eux, l'un desdits composants (3") formant entièrement ledit siège de positionnement (81).

18. Dispositif selon l'une quelconque des revendications 4 et 5, **caractérisé en ce que** ledit corps comprend au moins deux composants couplés entre eux, au moins l'un desdits composants (2', 3', 4' ; 2", 3", 4") faisant partie intégrante au moins dudit élément de raccordement.

19. Dispositif selon l"une quelconque des revendications 16 à 18, **caractérisé en ce que**
- lesdits composants (2', 3' ; 2", 3") sont couplés ensemble via un soudage ou par thermofusion ou collage, ou bien via des moyens de couplage mécaniques tels qu'un couplage d'un type à filetage femelle/filetage mâle, un couplage rapide, un couplage à baïonnette, un couplage par action d'encliquetage, et/ou
- lesdits composants (2', 3' ; 2", 3") sont assemblés au moins dans une région de couplage mutuel dans une zone ou paroi périphérique (2b, 3b) et/ou une zone ou paroi interne (3c, 3c).

20. Dispositif selon la revendication 1, **caractérisé en ce qu'**il comprend un connecteur électrique (CE) formé au moins en partie par ledit corps (2', 3' ; 2", 3") ou par un composant associé à ce dernier.

21. Dispositif selon la revendication 1, **caractérisé en ce qu'**il comprend un circuit électronique (60).

22. Dispositif selon la revendication 21, **caractérisé en ce que** ledit circuit électronique (60) comprend au moins l'un parmi :
- un circuit de mesure en particulier pour générer et/ou détecter des signaux électriques ;
- un processeur ou un microprocesseur numérique ou microcontrôleur ;
- des moyens de mémoire électronique ;
- un circuit d'amplification, de préférence d'un type programmable au moins pour le gain respectif ;
- un circuit pour le calibrage de type automatique ou de type conçu pour modifier une donnée numérique ou la valeur d'un composant, telle que par exemple une résistance ;
- un commutateur électronique ;
- un dispositif pour le contrôle ou l'actionnement ou la sélection ou la commutation ;
- un circuit d'alimentation en énergie électrique, et
- des moyens de communication, en particulier comprenant au moins l'un parmi une ligne de communication filaire des signaux et un circuit pour la communication sans fil des signaux.

23. Dispositif selon la revendication 8, **caractérisé en ce que** ladite électrode ou lesdites électrodes (40, 41 ; 40, 41, 42 ; 42, 44 ; 40' , 41') sont réalisées à partir de métal, en particulier obtenues à partir d'une bande de métal, de préférence de petite épaisseur.

24. Dispositif selon la revendication 8, **caractérisé en ce que** ladite électrode ou lesdites électrodes (40, 41 ; 40, 41, 42 ; 42, 44 ; 40', 41') sont formées au moins en partie avec un matériau synthétique qui est électriquement conducteur ou rendu électriquement conducteur tel qu'un matériau thermoplastique en ajoutant un matériau électriquement conducteur tel qu'une matière de remplissage, où en particulier ladite électrode ou lesdites électrodes (40, 41 ; 40, 41, 42 ; 42, 44 ; 40', 41') sont formées au moins en partie via le moulage ou une impression sérigraphique.

25. Dispositif selon la revendication 24, **caractérisé en ce que** ladite électrode ou lesdites électrodes (40', 41') comprennent un noyau électriquement conducteur (40e, 41e) recouvert au moins partiellement avec ledit matériau synthétique électriquement conducteur, en particulier surmoulé ou déposé.

26. Dispositif selon la revendication 24, **caractérisé en ce que** ledit matériau synthétique électriquement conducteur est surmoulé sur, ou réparti, ou sérigraphié sur au moins une partie d'un composant du dispositif lui-même, ou vice versa, ou co-moulé avec au moins une partie dudit composant, où en particulier un ou plusieurs sièges, canaux ou passages sont formés dans ledit composant (2) afin de recevoir ledit matériau synthétique électriquement conducteur et/ou des parties d'ancrage pour ce dernier.
